# EUROPEAN PATENT APPLICATION

(11) **EP 4 269 437 A1**
(43) Date of publication of application: **01.11.2023**
(21) Application number: 21914288.2
(22) Date of filing: 27.12.2021
(51) Int. Cl.: C07K 16/28, C12N 15/13, C12N 15/63, C12N 5/10, A61K 39/395, A61P 35/00, G01N 33/68

(54) **PD-1 BINDING MOLECULE AND APPLICATION THEREOF**

(30) Priority: 28.12.2020 CN 202011582908
(71) Applicant: Zhejiang Nanomab Technology Center Co. Ltd., Changle Township Shengzhou Shaoxing City Zhejiang Province, 312467 (CN)
(72) Inventor: LI, Jiaguo, Shanghai 201805 (CN); YU, Haixiang, Shanghai 201805 (CN); LIU, Xiangzhen, Shanghai 201805 (CN); ZHU, Weimin, Danville, California 94506 (US); SUN, Yan, Shanghai 201805 (CN); DING, Na, Shanghai 201805 (CN); QIAN, Qijun, Shanghai 201805 (CN)
(74) Representative: Cabinet Beau de Loménie
(86) International application number: PCT/CN2021/141740
(87) International publication number: WO 2022/143552

(57) **Abstract**

Provided by the present invention is a PD-1 binding molecule, which contains an anti-PD-1 single-domain antibody. The complementarity-determining region (CDR) of the single-domain antibody comprises CDR1 shown in SEQ ID NO:1, CDR2 shown in SEQ ID NO:2, and CDR3 shown in SEQ ID NO:3.

## Description

### Technical Field

The invention relates to the field of biomedicine or biopharmacy, more specifically to a PD-1 binding molecule and use thereof.

### Background

Programmed death receptor-1 protein, also known as PD-1 (programmed death 1), is a transmembrane protein that is expressed on the surface of T cells and function as an immunosuppressive. PD-1 has two ligand molecules, PD-L1 and PD-L2, respectively, which are expressed at higher levels on antigen-presenting cells. When PD-L1 and PD-L2 are combined with PD-1, they will negatively regulate the function and activity of T cells. Tumor cells are usually able to express PD-L1 or PD-L2 highly to achieve "immune escape" (Lee L. et al, J Clin Pharmacol. 2016 Feb;56(2):157-69). Therefore, PD-1 medicaments can kill tumors by positively regulating the immune system. Now, PD-1 is also famous for immune checkpoint inhibitor therapy.

The FDA approved two PD-1 monoclonal antibodies, Pembrolizumab (Keytruda) and Nivolumab (Opdivo), in 2014 and 2015, bases on their outstanding efficacy in the treatment of melanoma. The current indications have covered nearly 20 solid tumors (Prasad V et c. Semin Oncol. 2017 Apr;44(2): 132-135.). At present, four PD-1 mAb drugs have been lunched in China, namely Camrelizumab from Hengrui, Tislelizumab from BeiGene, Sintilizumab from Innovent and Toripalimab from Junshi.

Studies have found that traditional scfv express at a low level in T cells, and it is difficult to fully exert the effect of PD-1 inhibitors. This is mainly due to the characteristics of scfv, which is prone to aggregation, prone to mismatch when folding, and has low thermal stability ( Martin N et al, Macromol Biosci. 2017 Feb; 17(2)). Nanobodies have the advantages of easy expression, high stability and high affinity. According to the advantages of nanobodies and the biological mechanism of PD-1, the development of PD-1 nanobodies has very broad application prospects. It has become an urgent problem to develop a novel anti-PD-1 single domain antibody, which has good specificity, blocking activity, clinical efficacy, simple production, low cost, and reduced drug burden.

### Summary

The invention aims to provide a novel anti-PD-1 binding molecule and use thereof.

The first aspect of the invention provides a PD-1 binding molecule, which comprises an anti-PD-1 single domain antibody, wherein the complementarity determining region (CDR) of the single domain antibody comprises CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3.

In one or more embodiments, SEQ ID NO: 1 is GX₁X₂X₃X₄X₅X₆X₇, wherein X₁ is D, F, G, H, L or R, X₂ is P, SorT, and X₃ is A, F, I, L, N, R, S or V, X₄ is D, F, G, L, M, N or S, X₅ is A, D, F, G, I, N, R, S or T, X₆ is D, F, H, I, L, S, V, W or Y, X₇ is A, D, E, G, N, P, S or T. Exemplarily, CDR1 comprises the sequence shown in any one of SEQ ID NOs: 4-39,320.

Preferably, SEQ ID NO:1 is GX₁X₂X₃X₄X₅X₆X₇, wherein X₁ is D, F, G, H, L or R, X₂ is P, S or T, X₃ is F or S, X₄ is F or S, and X₅ is G, I or T, X₆ is S or Y, and X₇ is A or D. Exemplarily, CDR1 comprises the sequence shown in any one of SEQ ID NOs: 16, 19, 36-39, 320.

Preferably, SEQ ID NO:1 is GX₁X₂X₃X₄IX₆X₇, wherein X₁ is D or R, X₂ is P or T, X₃ is S or F, X₄ is F or S, X₆ is S or Y, X₇ is A or D. Exemplarily, CDR1 comprises the sequence shown in SEQ ID NOs: 16 or 19.

Alternatively, more preferably, SEQ ID NO:1 is GX₁X₂FSX₅YX₇, wherein X₁ is G, F, H, L or R, X₂ is P, S or T, X₅ is G, I or T, X₇ is A or D. Exemplarily, CDR1 comprises the sequence shown in any one of SEQ ID NOs: 19, 36-39, 320.

In one or more embodiments, SEQ ID NO: 2 is X₁X₂X₃X₄X₅X₆X₇X₈X₉, wherein X₁ is I, L, S, T or V, X₂ is A, N, SorT, X₃ is F, G, I, K, L, M, N, Q, R, S, T, W or Y, X₄ is A, D, G, H, N, R, SorT, X₅ is A, D, G, N, R, S or null, X₆ is G or R, SorT, X₇ is D, E, I, L, M, N, R, S, T or V, X₈ is A, K, M, Q or T, X₉ is A or null. Exemplarily, CDR2 comprises the sequence shown in any one of SEQ ID NOs: 40-75.

Preferably, SEQ ID NO:2 is X₁X₂X₃X₄X₅GX₇TX₉, wherein X₁ is I or V, X₂ is N, S or T, X₃ is F, L or N, X₄ is A, G, S or T, and X₅ is R or null, X₇ is D, N, I or T, and X₉ is null. Exemplarily, CDR2 comprises the sequence shown in any one of SEQ ID NOs: 52, 53, 55, 72-75.

More preferably, SEQ ID NO:2 is IX₂X₃X₄X₅GX₇TX₉, wherein X₂ is N, S or T, X₃ is L or N, X₄ is A, S or T, and X₅ is R or null, X₇ is D, I or N, X₉ is null. Exemplarily, the CDR2 comprises the sequence shown in any one of SEQ ID NOs: 52, 53, 55, 72, 73.

Alternatively, more preferably, SEQ ID NO:2 is X₁X₂X₃X₄RGX₇TX₉, wherein X₁ is I or V, X₂ is N, S or T, X₃ is F or L, X₄ is A, G or S, X₇ is D, N or T, X₉ is null. Alternatively, the CDR2 comprises SEQ ID NO: 55 or a variant thereof, the variant comprises a change of one or more amino acids selected from the group consisting of: X₁ is mutated from I to V, X₂ is mutated from N to S or T, and X₃ is mutated from L to F, X₄ is mutated from S to A or G, and X₇ is mutated from D to N or T. Exemplarily, CDR2 comprises the sequence shown in any one of SEQ ID NOs: 53, 55, 72-75.

In one or more embodiments, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, E, G, N, R, S, T or V, X₂ is A, G, I, K, L, P, R, T or V, X₃ is A, D, E, G, K, N, P, R, S, V or Y, X₄ is A, C, D, E, G, I, K, L, R, S, T, V or Y, X₅ is A, C, D, F, G, H, I, K, L, N, P, Q, R, S, W or Y, X₆ is A, C, D, E, G, I, K, L, M, P, Q, R, S or Y, X₇ is A, C, D, F, G, H, I, N, P, R, S, T, V or Y, X₈ is A, C, D, E, G, I, R, S, T, V, W, Y or null, X₉ is A, D, E, F, G, I, L, P, R, S, V, W, Y or null, X₁₀ is C, D, F, G, K, L, N, P, R, S, T, V, W, Y or null, X₁₁ is D, F, G , H, I, K, L, N, P, S, T, V, Y or null, X₁₂ is A, D, G, H, I, L, M, P, R, S, T, V, Y or null, X₁₃ is C, D, E, H, P, R, S, T, V, Y or null, X₁₄ is A, D, E, F, H, I, L, P, R, T, Y or null, X₁₅ is A, D, E, G, N, Q, R, S, T, V, Y or null, X₁₆ is A, D, E, F, G, H, M, P, S, V , Y or null, X₁₇ is A, D, E, H, N, Q, R, T, Y or null, X₁₈ is D, E, R, S, Y or null, X₁₉ is N, S, Y or null, X₂₀ is D, E, Y or null, X₂₁ is E, N, Y or null, X₂₂ is Y or null. Exemplarily, CDR3 comprises the sequence shown in any one of SEQ ID NOs: 76-183.

Preferably, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is E, G, N or V, X₂ is A, G, I, L or V, X₃ is A, D, E, G, N, V or Y, X₄ is A, G, I, K, R or S, X₅ is C, I, L, P, Q, R or W, X₆ is E, K, P, Q or R, X₇ is C, D, H, N, P or Y, X₈ is E, G, R, V or W, X₉ is F, G, L, S, V or W, X₁₀ is C, D, G, L, R, S or V, X₁₁ is F, G, I, L, N, S, T or V, X₁₂ is H, L, P, R, S, T or Y, X₁₃ is D, H, P, S, T or V, X₁₄ is E, F, H, I, L, P or R, X₁₅ is A, S or V, X₁₆ is A, D, E, G, H or V, X₁₇ is H, N, Q, R or Y, X₁₈-X₂₂ is null. Exemplarily, CDR3 comprises the sequence shown in any one of SEQ ID NOs: 76, 87, 108-183.

More preferably, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is E, G or V, X₂ is A, G, I, L or V, X₃ is A, D, E, G, N, V or Y, X₄ is G, I, K, R or S, X₅ is C, I, L, Q, R or W, X₆ is E, K, P, Q or R, X₇ is C, D, H, N or Y, X₈ is E, G, R, V or W, X₉ is F, L, S, V or W, X₁₀ is C, D, G, R, S or V, X₁₁ is F, I, L, N, S, T or V, X₁₂ is H, L, P, R, SorT, X₁₃ is H, P, S, T or V, X₁₄ is F, H, I, L, P or R, X₁₅ is A, S or V, X₁₆ is A, D, E, G, H or V, X₁₇ is H, N, Q, R or Y, X₁₈-X₂₂ is null. Alternatively, the CDR3 comprises the sequence shown in SEQ ID NO: 87 or variants thereof, the variants comprise one or more amino acid changes selected from the group consisting of: the G of X₁ is mutated to E, G or V, the V of X₂ is mutated to A, G, I or V, the D of X₃ is mutated to A, E, G, N, V or Y, the R of X₄ is mutated to G, I, K or S, the R of X₅ is mutated to C, I, L, Q or W, the Q of X₆ is mutated to E, K, P or R, the Y of X₇ is mutated to C, D, H or N, the G of X₈ is mutated to E, R, V or W, and the L of X₉ is mutated to F, S , V or W, the G of X₁₀ is mutated to C, D, R, S or V, the I of X₁₁ is mutated to F, L, N, S, T or V, and the P of X₁₂ is mutated to H, L, R, S or T, the P of X₁₃ is mutated to H, S, T or V, the L of X₁₄ is mutated to F, H, I, P or R, the A of X₁₅ is mutated to S or V, the D of X₁₆ is mutated to A, E, G, H or V, the H of X₁₇ is mutated to N, Q, R or Y. Exemplarily, CDR3 comprises the sequence shown in any one of SEQ ID NOs: 87, 108-183.

In one or more embodiments, CDR1 comprises the sequence shown in any one of SEQ ID NOs: 4-39 and 320, CDR2 comprises the sequence shown in any one of SEQ ID NOs: 40-75, and CDR3 comprises the sequence shown in any one of SEQ ID NOs: 76-183.

In one or more embodiments, CDR1 comprises the sequence shown in any one of SEQ ID NOs: 16-39 and 320, CDR2 comprises the sequence shown in any one of SEQ ID NOs: 52-75, and CDR3 comprises the sequence shown in any one of SEQ ID NOs: 76, 87, 108-183.

In one or more embodiments, CDR1 comprises the sequence shown in any one of SEQ ID NOs: 16 or 19, CDR2 comprises the sequence shown in any one of SEQ ID NOs: 52, 53, 55, 72, 73, and CDR3 comprises the sequence shown in any one of SEQ ID NOs: 87, 108-111, 113-134, 136-146, 148-154, 156-163, 165-168, 172, 173, 177-183.

In one or more embodiments, CDR1 comprises the sequence shown in any one of SEQ ID NOs: 19, 36-39 and 320, CDR2 comprises the sequence shown in any one of SEQ ID NOs: 53, 55, 72-75, and CDR3 comprises the sequence shown in any one of SEQ ID NOs: 87, 108-183.

In one or more embodiments, CDR1 comprises the sequence shown in any one of SEQ ID NOs: 5, 7, 10, 14-16, 19, 26, 27, 29, 30, 33, CDR2 comprises the sequence shown in any one of SEQ ID NOs: 41, 47, 50-52, 55, 56, 61-66, and CDR3 comprises the sequence shown in any one of SEQ ID NOs: 76, 77, 85-87, 93, 94, 97, 99-101, 104.

In one or more embodiments, the single domain antibody comprises CDR1, CDR2, and CDR3 shown in any one of the following groups a1 to a136:

| CDR1 | CDR2 | CDR3 | Group |
|---|---|---|---|
| 4 | 40 | 76 | a1 |
| 5 | 41 | 77 | a2 |
| 6 | 42 | 78 | a3 |
| 7 | 43 | 76 | a4 |
| 8 | 44 | 79 | a5 |
| 9 | 45 | 80 | a6 |
| 10 | 46 | 81 | a7 |
| 11 | 47 | 82 | a8 |
| 12 | 48 | 83 | a9 |
| 13 | 49 | 84 | a10 |
| 10 | 50 | 85 | a11 |
| 14 | 47 | 76 | a12 |
| 15 | 51 | 86 | a13 |
| 16 | 52 | 76 | a14 |
| 17 | 53 | 87 | a15 |
| 18 | 54 | 88 | a16 |
| 19 | 55 | 87 | a17 |
| 20 | 56 | 82 | a18 |
| 21 | 57 | 89 | a19 |
| 22 | 58 | 90 | a20 |
| 23 | 59 | 91 | a21 |
| 14 | 47 | 76 | a22 |
| 24 | 60 | 92 | a23 |
| 25 | 40 | 76 | a24 |
| 26 | 61 | 93 | a25 |
| 27 | 62 | 94 | a26 |
| 24 | 60 | 95 | a27 |
| 28 | 41 | 96 | a28 |
| 10 | 63 | 97 | a29 |
| 5 | 41 | 98 | a30 |
| 29 | 64 | 99 | a31 |
| 7 | 65 | 100 | a32 |
| 30 | 66 | 101 | a33 |
| 31 | 67 | 102 | a34 |
| 32 | 68 | 103 | a35 |
| 33 | 56 | 104 | a36 |
| 34 | 69 | 105 | a37 |
| 35 | 70 | 106 | a38 |
| 7 | 71 | 107 | a39 |
| 16 | 52 | 76 | a40 |
| 16 | 52 | 76 | a41 |
| 16 | 52 | 76 | a42 |
| 16 | 52 | 76 | a43 |
| 16 | 52 | 76 | a44 |
| 19 | 72 | 87 | a45 |
| 19 | 72 | 87 | a46 |
| 19 | 72 | 87 | a47 |
| 19 | 73 | 87 | a48 |
| 19 | 53 | 87 | a49 |
| 19 | 53 | 87 | a50 |
| 19 | 53 | 108 | a51 |
| 19 | 53 | 109 | a52 |
| 19 | 53 | 110 | a53 |
| 19 | 53 | 111 | a54 |
| 36 | 74 | 112 | a55 |
| 19 | 53 | 113 | a56 |
| 19 | 53 | 114 | a57 |
| 19 | 53 | 115 | a58 |
| 19 | 53 | 116 | a59 |
| 19 | 53 | 117 | a60 |
| 19 | 53 | 118 | a61 |
| 19 | 53 | 119 | a62 |
| 19 | 53 | 120 | a63 |
| 19 | 53 | 121 | a64 |
| 19 | 53 | 122 | a65 |
| 19 | 53 | 123 | a66 |
| 19 | 53 | 124 | a67 |
| 19 | 53 | 125 | a68 |
| 19 | 53 | 126 | a69 |
| 19 | 53 | 127 | a70 |
| 19 | 53 | 128 | a71 |
| 19 | 53 | 129 | a72 |
| 19 | 53 | 130 | a73 |
| 19 | 53 | 131 | a74 |
| 19 | 53 | 132 | a75 |
| 19 | 53 | 133 | a76 |
| 19 | 53 | 134 | a77 |
| 37 | 75 | 135 | a78 |
| 19 | 53 | 136 | a79 |
| 19 | 53 | 137 | a80 |
| 19 | 53 | 138 | a81 |
| 19 | 53 | 139 | a82 |
| 19 | 53 | 140 | a83 |
| 19 | 53 | 141 | a84 |
| 19 | 53 | 142 | a85 |
| 19 | 53 | 143 | a86 |
| 19 | 53 | 144 | a87 |
| 19 | 53 | 145 | a88 |
| 19 | 53 | 146 | a89 |
| 38 | 53 | 147 | a90 |
| 19 | 53 | 148 | a91 |
| 19 | 53 | 149 | a92 |
| 19 | 53 | 150 | a93 |
| 19 | 53 | 151 | a94 |
| 19 | 53 | 152 | a95 |
| 19 | 53 | 153 | a96 |
| 19 | 53 | 154 | a97 |
| 39 | 53 | 155 | a98 |
| 19 | 53 | 156 | a99 |
| 19 | 53 | 157 | a100 |
| 19 | 53 | 158 | a101 |
| 19 | 53 | 159 | a102 |
| 19 | 53 | 160 | a103 |
| 19 | 53 | 161 | a104 |
| 19 | 53 | 162 | a105 |
| 19 | 53 | 163 | a106 |
| 37 | 53 | 164 | a107 |
| 19 | 53 | 165 | a108 |
| 19 | 53 | 166 | a109 |
| 19 | 53 | 167 | a110 |
| 19 | 53 | 168 | a111 |
| 36 | 74 | 169 | a112 |
| 38 | 53 | 170 | a113 |
| 37 | 53 | 171 | a114 |
| 19 | 53 | 172 | a115 |
| 19 | 53 | 173 | a116 |
| 38 | 53 | 174 | a117 |
| 38 | 53 | 175 | a118 |
| 36 | 74 | 176 | a119 |
| 19 | 53 | 177 | a120 |
| 19 | 53 | 178 | a121 |
| 19 | 53 | 179 | a122 |
| 19 | 53 | 180 | a123 |
| 19 | 53 | 181 | a124 |
| 19 | 53 | 182 | a125 |
| 19 | 53 | 183 | a126 |
| 37 | 75 | 135 | a127 |
| 37 | 75 | 135 | a128 |
| 37 | 75 | 135 | a129 |
| 37 | 75 | 135 | a130 |
| 37 | 75 | 135 | a131 |
| 37 | 75 | 135 | a132 |
| 37 | 75 | 135 | a133 |
| 37 | 75 | 135 | a134 |
| 320 | 75 | 135 | a135 |
| 37 | 75 | 135 | a136 |

In one or more embodiments, FR1 of the single domain antibody VHH can be selected from the FR1 of VHH of each antibody numbering in Table 1, FR2 of VHH can be selected from the FR2 of VHH of each antibody numbering in Table 1, FR3 of VHH can be selected from the FR3 of VHH of each antibody numbering in Table 1, and FR4 of VHH can be selected from the FR4 of VHH of each antibody numbering in Table 1.

In one or more embodiments, the FR region of the single domain antibody is the FR region of any VHH selected from SEQ ID NOs: 184-319.

In one or more embodiments, the single domain antibody VHH is as shown in any one of SEQ ID NOs: 184-319. Preferably, the single domain antibody is as shown in any one of SEQ ID NOs: 197, 200, 223-319.

In one or more embodiments, the PD-1 binding molecule is a monovalent or multivalent single domain antibody, a multispecific single domain antibody, a heavy chain antibody or an antigen binding fragment thereof, an antibody or an antigen binding fragment thereof comprising one, two or more of the anti-PD-1 single domain antibodies described herein.

In one or more embodiments, the multivalent single domain antibody or multispecific single domain antibody connects a plurality of single domain antibodies through a linker. The linker consists of 1-15 amino acids selected from G and S.

In one or more embodiments, the antigen binding fragment of the heavy chain antibody is a single chain heavy chain antibody.

In one or more embodiments, the heavy chain antibody is a camelid heavy chain antibody or a shark heavy chain antibody.

In one or more embodiments, the heavy chain antibody further comprises a heavy chain constant region.

In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG4. Preferably, the heavy chain constant region is shown in SEQ ID NO: 321.

In one or more embodiments, the heavy chain constant region is a constant region of the shark heavy chain antibody, comprising CH1, CH2, CH3, CH4, and CH5.

In one or more embodiments, the antibody is an antibody comprising the anti-PD-1 single domain antibody as the heavy chain variable domain.

In one or more embodiments, the antibody further comprises a light chain variable domain, a heavy chain constant domain, and a light chain constant domain.

In one or more embodiments, the antigen binding fragment of the antibody is selected from Fab, F(ab')2, Fv, scFv.

In one or more embodiments, the binding molecule described in any embodiment of the present description is a chimeric antibody or a fully human antibody; preferably, a fully human antibody.

The description also provides a polynucleotide, comprising a sequence selected from:
(1) a coding sequence of the single domain antibody or the antibody or the antigen binding fragment thereof according to any embodiment herein;
(2) a complementary sequence of (1);
(3) a 5-50bp fragment of any sequence of (1) or (2).

In one or more embodiments, the fragment is primer.

In one or more embodiments, the sequence of the polynucleotide comprises the sequence shown in SEQ ID NO: 322 or 323.

The description also provides a nucleic acid construct comprising the polynucleotide described herein.

In one or more embodiments, the nucleic acid construct is a recombinant vector or expression vector.

The description also provides a phage comprising the PD-1 binding molecule according to any embodiment herein.

In one or more embodiments, the PD-1 binding molecule is displayed on the surface of the phage.

The description also provides a host cell selected from:
(1) the host cell expressing the PD-1 binding molecule according to any embodiment herein;
(2) the host cell comprising a polynucleotide described herein; and/or
(3) the host cell comprising a nucleic acid construct described herein.

The description also provides a method for producing a PD-1 binding molecule, comprising culturing the host cells described herein under conditions suitable for producing the PD-1 binding molecule (such as monovalent or multivalent single domain antibodies, multispecific single domain antibodies, heavy chain antibodies, antibodies or antigen binding fragments thereof), and optionally purifying the PD-1 binding molecule from culture.

The description also provides a pharmaceutical composition, comprising the PD-1 binding molecule, polynucleotide, nucleic acid construct, phage or host cell according to any embodiment herein, and a pharmaceutically acceptable excipient.

In one or more embodiments, the pharmaceutical composition is used for treating cancer.

In one or more embodiments, the cancer is PD-1 related cancer. Preferably, the cancer is selected from the group consisting of: melanoma, lung cancer, head and neck cancer, renal cell carcinoma, urothelial carcinoma, non-Hodgkin's lymphoma, and the like.

The description also provides use of the PD-1 binding molecule according to any embodiment herein in the preparation of a medicament for the prevention or treatment of a cancer.

In one or more embodiments, the cancer is a PD-1 related cancer. Preferably, the cancer is selected from the group consisting of: melanoma, lung cancer, head and neck cancer, renal cell carcinoma, urothelial carcinoma, non-Hodgkin's lymphoma, and the like. The description also provides a method for treating or preventing a cancer, comprising administrating a patient in need thereof an effective amount of a PD-1 binding molecule according to any embodiment of the description, or a pharmaceutical composition comprising a PD-1 binding molecule according to any embodiment of the description.

In one or more embodiments, the cancer is a PD-1 related cancer. Preferably, the cancer is selected from the group consisting of: melanoma, lung cancer, head and neck cancer, renal cell carcinoma, urothelial carcinoma, non-Hodgkin's lymphoma, and the like.

The description also provides a kit for detecting PD-1, for use in evaluating the therapeutic effect of a medicament or diagnosing cancer. The kit comprises a PD-1 binding molecule, polynucleotide, nucleic acid construct, phage or host cell according to any embodiment of the description.

In one or more embodiments, the kit further comprises a reagent for detecting the binding of PD-1 to a single domain antibody, an antibody, or an antigen binding fragment thereof. For example, the bound reagent is detected by the enzyme-linked immunosorbent assay.

In one or more embodiments, the detection reagent for binding is a detectable marker, such as biotin, that can be linked to a PD-1 binding molecule. The detectable marker is connected to the PD-1 binding molecule or present in the kit separately.

The description also provides a non diagnostic method for detecting the presence of PD-1 in a sample. The method comprises: incubating a PD-1 binding molecule according to any embodiment herein with the sample, and detecting the binding of PD-1 to a single domain antibody, antibody, or antigen binding fragment thereof, thereby determining the presence of PD-1 in the sample. The detection is an enzyme-linked immunosorbent assay.

The description also provides use of a PD-1 binding molecule according to any embodiment herein in the preparation of a kit for detecting PD-1 in a sample, evaluating the therapeutic effect of a medicament or diagnosing a cancer.

### Description of Figures

Figure 1 shows the detection of titer of Alpaca antiserum against PD-1 protein.
Figure 2 shows the detection of titer of Alpaca antiserum against PD-1 overexpressing cell line.
Figure 3 shows activation of luciferase reporter gene by anti-PD-1 antibody. Isotype control (negative control), and its amino acid sequence is shown in SEQ ID NO: 2 in CN106046152A.
Figure 4 shows the effect of PD-1 antibody on IFN-γ secretion in mixed lymphocyte reaction (MLR). Isotype control is the same as Figure 3.
Figure 5 shows the effect of PD-1 antibody on IL-2 secretion in mixed lymphocyte reaction (MLR). Isotype control is the same as Figure 3.
Figure 6 shows the cross-reaction results of NB 182 antibody with 5300 membrane proteins.
Figure 7 shows the cross-reaction results of NB 194 antibody with 5300 membrane proteins.
Figure 8 shows that the NB194 mutant also has improved affinity and functional activity. EC50 is present in nM. ∘

### Detailed Description

After extensive and in-depth research and a large number screening, the inventors finds a class of PD-1 binding molecules comprising anti-PD-1 single-domain antibodies. The experimental results show that the binding molecules of the description could specifically recognize the PD-1, binds to PD-1 with high affinity, has good functional activity, can promote T cells to secrete cytokines, and has no tissue cross-reactivity. The single domain antibody of the description is simple to generate.

Specifically, the invention immunizes Alpaca with human PD-1 protein to obtain a high-quality immune single domain antibody gene library. Then the PD-1 protein is coupled on a microtiter plate, and the immune single domain antibody gene library is screened by phage display technology, so as to obtain the PD-1 specific single domain antibody gene. Then the gene was transferred to mammalian cells, and a single domain antibody strain with high specificity and high expression in mammalian cells is obtained. Then, single domain antibodies with high affinity, high specificity, high functional activity and low tissue cross-reactivity were identified by ELISA, flow cytometry, membrane protein cross-reactivity, epitope competition assay and other methods.

### Antibody

"PD-1 binding molecule" as used herein is a protein that specifically binds PD-1, including but not limited to antibodies, antigen binding fragments of antibodies, heavy chain antibodies, nanobodies, micro antibodies, affibodies, target binding regions of receptors, cell adhesion molecules, ligands, enzymes, cytokines, and chemokines.

Term "antibody" as used herein includes monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and FV). The term "immunoglobulin" (Ig) is used interchangeably with "antibody" herein.

The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblages. In the case of IgGs, the 4-chain unit is typically about 150,000 daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α , δ, ε, γ and µ, respectively. The γ and α classes are further divided into subclasses on the basis of relatively minor differences in the CH sequence and function, e.g., humans express the following subclasses: IgGl, IgG2A, IgG2B, IgG3, IgG4, IgA1 and IgA2.

"Heavy chain antibody" described herein is an antibody derived from camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is connected to the constant region through a hinge region like structure. Each heavy chain of camelid heavy chain antibody contains one variable region (VHH) and two constant regions (CH2 and CH3), and each heavy chain of shark heavy chain antibody contains one variable region and five constant regions (CH1-CH5). Antigen binding fragments of heavy chain antibodies include VHH and single chain heavy chain antibodies. Heavy chain antibodies may have CH2 and CH3 of human IgG Fc by fusing with the constant region of human IgG Fc.

As used herein, the terms "single domain antibody", "anti-PD-1 single domain antibody", "heavy chain variable region domain of heavy chain antibody", "VHH" and "nanobody" can be used interchangeably, and all refer to single domain antibodies that specifically recognize and bind to PD-1. Single domain antibodies are variable regions of heavy chain antibodies. Typically, single domain antibodies contain three CDRs and four FRs. Preferably, the single domain antibody of the description has CDR1 shown in SEQ ID NO: 1, CDR2 shown in SEQ ID NO: 2, and CDR3 shown in SEQ ID NO: 3. Single domain antibodies are the smallest functional antigen binding fragments. Generally, after obtaining an antibody which naturally lacks light chain and heavy chain constant region 1 (CH1), the variable region of the heavy chain of the antibody is cloned to construct a single domain antibody consisting of only one heavy chain variable region.

Binding molecules comprising two or more single domain antibodies are multivalent single domain antibodies; and binding molecules comprising two or more single domain antibodies with different specificities are multispecific single domain antibodies. Multivalent single domain antibodies or multispecific single domain antibodies are connected to multiple single domain antibodies through linkers. The linker usually consists of 1-15 amino acids selected from G and S.

The terms "heavy chain antibody" and "antibody" herein are intended to distinguish different composition forms of antibodies. Due to the similarity of their structures, the following descriptions on structures of antibodies except for light chains also apply to heavy chain antibodies.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains, namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved portions of variable domains are called the framework regions (FR). The variable domains of native heavy and light chains each comprise four FR regions (FR1, FR2, FR3, and FR4), largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domains are not involved directly in the binding of antibody to an antigen, but exhibit various effector functions, such as participation of the antibody in antibody-dependent cellular toxicity.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. In IgG, IgA and IgD antibody isotypes, the Fc region is composed of two identical protein fragments, derived from CH2 and CH3 constant domains of the antibody's two heavy chains; IgM and IgE Fc regions contain three heavy chain constant domains (CH domains 2-4) in each polypeptide chain. Although the boundaries of the Fc region of an immunoglobulin heavy chain might vary, the human IgG heavy chain Fc region is usually defined to stretch from an amino acid residue at position C226 or P230 to the carboxy-terminus of the heavy chain, wherein the numbering is according to the EU index as in Kabat. As used herein, the Fc region may be a native sequence Fc or a variant Fc.

An "antibody fragment" comprises a portion of an intact antibody, preferably the antigen binding and/or the variable region of the intact antibody. The antibody fragment is preferably an antigen binding fragment of the antibody. Examples of antibody fragments include Fab, Fab', F(ab')2 and Fv fragments; diabodies; linear antibodies; single-chain antibody molecules, scFv-Fc fragment; multispecific antibodies formed from antibody fragments; and any fragment that should be able to increase the half-life by chemical modification or by incorporation into liposomes. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. F(ab')2 antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method, phage-display technologies, recombinant DNA methods, and technologies for producing human or humanlike antibodies in animals that have parts or all of the human immunoglobulin loci or genes encoding human immunoglobulin sequences, single-cell sequencing methods.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable- domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies are well known in the art, such as using mice with genetically engineered immune systems. In the description, antibodies, single domain antibodies, heavy chain antibodies, etc. all include humanized variants of the antibodies.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries.

In some embodiments, the description also provides a single domain antibody, heavy chain antibody, antibody or antigen binding fragment thereof that binds to the same epitope of PD-1 as any anti-PD-1 single domain antibody of the description, that is, a single domain antibody, heavy chain antibody, antibody or antigen binding fragment thereof that can cross-compete with any single domain antibody of the description for binding to PD-1.

In the present invention, the CDR1 of the anti-PD-1 single domain antibody comprises the sequence shown in SEQ ID NO: 1, and SEQ ID NO: 1 is GX₁X₂X₃X₄X₅X₆X₇, wherein X₁ is D, F, G, H, L or R, X₂ is P, S or T, and X₃ is A, F, I, L , N, R, S or V, X₄ is D, F, G, L, M, N or S, X₅ is A, D, F, G, I, N, R, SorT, X₆ is D, F, H, I, L, S, V, W or Y, X₇ is A, D, E, G, N, P, S or T. Exemplarily, CDR1 comprises the sequence shown in any one of SEQ ID NOs: 4-39,320.

The CDR2 of the anti-PD-1 single domain antibody comprises the sequence shown in SEQ ID NO: 2, SEQ ID NO: 2 is X₃X₂X₃X₄X₅X₆X₇X₈X₉, wherein X₁ is I, L, S, T or V, X₂ is A, N, SorT, X₃ is F, G, I, K, L, M, N, Q, R, S, T, W or Y, X₄ is A, D, G, H, N, R, S or T, X₅ is A, D, G, N, R, S or null, X₆ is G or R, SorT, X₇ is D, E, I, L, M, N, R, S, T or V, X₈ is A, K, M, Q or T, X₉ is A or null. Exemplarily, CDR2 comprises the sequence shown in any one of SEQ ID NOs: 40-75.

The CDR3 of the anti-PD-1 single domain antibody comprises the sequence shown in SEQ ID NO: 3, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, E, G, N, R, S, T or V, X₂ is A, G, I, K, L, P, R, T or V, X₃ is A, D, E, G, K, N, P, R, S, V or Y, X₄ is A, C, D, E, G, I, K, L, R, S, T, V or Y, X₅ is A, C, D, F, G, H, I, K, L, N, P, Q, R, S, W or Y, X₆ is A, C, D, E, G, I, K, L, M, P, Q, R, S or Y, X₇ is A, C, D, F, G, H, I, N, P, R, S, T, V or Y, X₈ is A, C, D, E, G, I, R, S, T, V, W, Y or null, X₉ is A, D, E, F, G, I, L, P, R, S, V, W, Y or null, X₁₀ is C, D, F, G, K, L, N, P, R, S, T, V, W, Y or null, X₁₁ is D, F, G, H, I, K, L, N, P, S, T, V, Y or null, X₁₂ is A, D, G, H, I, L, M, P, R, S, T, V, Y or null, X₁₃ is C, D, E, H, P, R, S, T, V, Y or null, X₁₄ is A, D, E, F, H, I, L, P, R, T, Y or null, X₁₅ is A, D, E, G, N, Q, R, S, T, V, Y or null, X₁₆ is A, D, E, F, G, H, M, P, S, V , Y or null, X₁₇ is A, D, E, H, N, Q, R, T, Y or null, X₁₈ is D, E, R, S, Y or null, X₁₉ is N, S, Y or null, X₂₀ is D, E, Y or null, X₂₁ is E, N, Y or null, X₂₂ is Y or null. Exemplarily, CDR3 comprises the sequence shown in any one of SEQ ID NOs: 76-183.

In one or more embodiments, the single domain antibody is NB182, NB194 or a variant thereof, CDR1 comprises the sequence shown in any one of SEQ ID NOs: 16, 19, 36-39 and 320, CDR2 comprises the sequence shown in any one of SEQ ID NOs: 52, 53, 55, 72-75, and CDR3 comprises the sequence shown in any one of SEQ ID NOs: 76, 87, 108-183. Alternatively, CDR1 comprises the sequence shown in any one of SEQ ID NOs: 16 or 19, CDR2 comprises the sequence shown in any one of SEQ ID NOs: 52, 53, 55, 72, 73, and CDR3 comprises the sequence shown in any one of SEQ ID NOs: 87, 108-111, 113-134, 136-146, 148-154, 156-163, 165-168, 172, 173, 177-183. Alternatively, CDR1 comprises the sequence shown in any one of SEQ ID NOs: 19, 36-39 and 320, CDR2 comprises the sequence shown in any one of SEQ ID NOs: 53, 55, 72-75, and CDR3 comprises the sequence shown in any one of SEQ ID NOs: 87, 108-183.

In one or more embodiments, the single domain antibody comprises CDR1, CDR2, and CDR3 shown in any of groups a1 to a136 in Table 1:

**Table 1**

| Antibody No. | CDR1 | CDR2 | CDR3 | VHH | Group |
|---|---|---|---|---|---|
| NB139 | 4 | 40 | 76 | 184 | a1 |
| NB 147 | 5 | 41 | 77 | 185 | a2 |
| NB148 | 6 | 42 | 78 | 186 | a3 |
| NB153 | 7 | 43 | 76 | 187 | a4 |
| NB154 | 8 | 44 | 79 | 188 | a5 |
| NB159 | 9 | 45 | 80 | 189 | a6 |
| NB 164 | 10 | 46 | 81 | 190 | a7 |
| NB 166 | 11 | 47 | 82 | 191 | a8 |
| NB169 | 12 | 48 | 83 | 192 | a9 |
| NB 170 | 13 | 49 | 84 | 193 | a10 |
| NB171 | 10 | 50 | 85 | 194 | a11 |
| NB 172 | 14 | 47 | 76 | 195 | a12 |
| NB 173 | 15 | 51 | 86 | 196 | a13 |
| NB182 | 16 | 52 | 76 | 197 | a14 |
| NB189 | 17 | 53 | 87 | 198 | a15 |
| NB 192 | 18 | 54 | 88 | 199 | a16 |
| NB 194 | 19 | 55 | 87 | 200 | a17 |
| NB195 | 20 | 56 | 82 | 201 | a18 |
| NB 196 | 21 | 57 | 89 | 202 | a19 |
| NB 197 | 22 | 58 | 90 | 203 | a20 |
| NB200 | 23 | 59 | 91 | 204 | a21 |
| NB202 | 14 | 47 | 76 | 205 | a22 |
| NB205 | 24 | 60 | 92 | 206 | a23 |
| NB209 | 25 | 40 | 76 | 207 | a24 |
| NB211 | 26 | 61 | 93 | 208 | a25 |
| NB215 | 27 | 62 | 94 | 209 | a26 |
| NB216 | 24 | 60 | 95 | 210 | a27 |
| NB218 | 28 | 41 | 96 | 211 | a28 |
| NB220 | 10 | 63 | 97 | 212 | a29 |
| NB221 | 5 | 41 | 98 | 213 | a30 |
| NB222 | 29 | 64 | 99 | 214 | a31 |
| NB223 | 7 | 65 | 100 | 215 | a32 |
| NB532 | 30 | 66 | 101 | 216 | a33 |
| NB548 | 31 | 67 | 102 | 217 | a34 |
| NB733 | 32 | 68 | 103 | 218 | a35 |
| NB734 | 33 | 56 | 104 | 219 | a36 |
| NB735 | 34 | 69 | 105 | 220 | a37 |
| NB754 | 35 | 70 | 106 | 221 | a38 |
| NB755 | 7 | 71 | 107 | 222 | a39 |
| NB 182-z 1 | 16 | 52 | 76 | 223 | a40 |
| NB 182-z2 | 16 | 52 | 76 | 224 | a41 |
| NB 182-z3 | 16 | 52 | 76 | 225 | a42 |
| NB182-z4 | 16 | 52 | 76 | 226 | a43 |
| NB 182-z5 | 16 | 52 | 76 | 227 | a44 |
| NB194-z1 | 19 | 72 | 87 | 228 | a45 |
| NB194-z2 | 19 | 72 | 87 | 229 | a46 |
| NB194-z3 | 19 | 72 | 87 | 230 | a47 |
| NB194-z4 | 19 | 73 | 87 | 231 | a48 |
| NB194-z5 | 19 | 53 | 87 | 232 | a49 |
| NB194-P0 1 | 19 | 53 | 87 | 233 | a50 |
| NB194-P0 2 | 19 | 53 | 108 | 234 | a51 |
| NB194-P0 3 | 19 | 53 | 109 | 235 | a52 |
| NB194-P0 4 | 19 | 53 | 110 | 236 | a53 |
| NB194-P0 5 | 19 | 53 | 111 | 237 | a54 |
| NB194-P0 6 | 36 | 74 | 112 | 238 | a55 |
| NB194-P0 7 | 19 | 53 | 113 | 239 | a56 |
| NB194-P0 8 | 19 | 53 | 114 | 240 | a57 |
| NB194-P0 9 | 19 | 53 | 115 | 241 | a58 |
| NB194-P1 0 | 19 | 53 | 116 | 242 | a59 |
| NB194-P1 1 | 19 | 53 | 117 | 243 | a60 |
| NB194-P1 2 | 19 | 53 | 118 | 244 | a61 |
| NB 194-P 1 3 | 19 | 53 | 119 | 245 | a62 |
| NB 194-P 1 4 | 19 | 53 | 120 | 246 | a63 |
| NB 194-P 1 5 | 19 | 53 | 121 | 247 | a64 |
| NB 194-P 1 6 | 19 | 53 | 122 | 248 | a65 |
| NB194-P1 7 | 19 | 53 | 123 | 249 | a66 |
| NB 194-P 1 8 | 19 | 53 | 124 | 250 | a67 |
| NB194-P1 9 | 19 | 53 | 125 | 251 | a68 |
| NB 194-P2 0 | 19 | 53 | 126 | 252 | a69 |
| NB194-P2 1 | 19 | 53 | 127 | 253 | a70 |
| NB194-P2 2 | 19 | 53 | 128 | 254 | a71 |
| NB 194-P2 3 | 19 | 53 | 129 | 255 | a72 |
| NB 194-P2 4 | 19 | 53 | 130 | 256 | a73 |
| NB 194-P2 5 | 19 | 53 | 131 | 257 | a74 |
| NB194-P2 6 | 19 | 53 | 132 | 258 | a75 |
| NB194-P2 7 | 19 | 53 | 133 | 259 | a76 |
| NB194-P2 8 | 19 | 53 | 134 | 260 | a77 |
| NB194-P2 9 | 37 | 75 | 135 | 261 | a78 |
| NB 194-P3 0 | 19 | 53 | 136 | 262 | a79 |
| NB 194-P3 1 | 19 | 53 | 137 | 263 | a80 |
| NB 194-P3 2 | 19 | 53 | 138 | 264 | a81 |
| NB 194-P3 3 | 19 | 53 | 139 | 265 | a82 |
| NB 194-P3 4 | 19 | 53 | 140 | 266 | a83 |
| NB 194-P3 5 | 19 | 53 | 141 | 267 | a84 |
| NB 194-P3 6 | 19 | 53 | 142 | 268 | a85 |
| NB 194-P3 7 | 19 | 53 | 143 | 269 | a86 |
| NB 194-P3 8 | 19 | 53 | 144 | 270 | a87 |
| NB 194-P3 9 | 19 | 53 | 145 | 271 | a88 |
| NB 194-P4 0 | 19 | 53 | 146 | 272 | a89 |
| NB 194-P4 1 | 38 | 53 | 147 | 273 | a90 |
| NB 194-P4 2 | 19 | 53 | 148 | 274 | a91 |
| NB 194-P4 3 | 19 | 53 | 149 | 275 | a92 |
| NB 194-P4 4 | 19 | 53 | 150 | 276 | a93 |
| NB 194-P4 5 | 19 | 53 | 151 | 277 | a94 |
| NB 194-P4 6 | 19 | 53 | 152 | 278 | a95 |
| NB 194-P4 7 | 19 | 53 | 153 | 279 | a96 |
| NB 194-P4 8 | 19 | 53 | 154 | 280 | a97 |
| NB 194-P4 9 | 39 | 53 | 155 | 281 | a98 |
| NB194-P5 0 | 19 | 53 | 156 | 282 | a99 |
| NB194-P5 1 | 19 | 53 | 157 | 283 | a100 |
| NB194-P5 2 | 19 | 53 | 158 | 284 | a101 |
| NB194-P5 3 | 19 | 53 | 159 | 285 | a102 |
| NB194-P5 4 | 19 | 53 | 160 | 286 | a103 |
| NB194-P5 5 | 19 | 53 | 161 | 287 | a104 |
| NB194-P5 6 | 19 | 53 | 162 | 288 | a105 |
| NB194-P5 7 | 19 | 53 | 163 | 289 | a106 |
| NB 194-P5 8 | 37 | 53 | 164 | 290 | a107 |
| NB194-P5 9 | 19 | 53 | 165 | 291 | a108 |
| NB 194-P6 0 | 19 | 53 | 166 | 292 | a109 |
| NB 194-P6 1 | 19 | 53 | 167 | 293 | a110 |
| NB 194-P6 2 | 19 | 53 | 168 | 294 | a111 |
| NB 194-P6 3 | 36 | 74 | 169 | 295 | a112 |
| NB 194-P6 4 | 38 | 53 | 170 | 296 | a113 |
| NB 194-P6 5 | 37 | 53 | 171 | 297 | a114 |
| NB 194-P6 6 | 19 | 53 | 172 | 298 | a115 |
| NB 194-P6 7 | 19 | 53 | 173 | 299 | a116 |
| NB 194-P6 8 | 38 | 53 | 174 | 300 | a117 |
| NB 194-P6 9 | 38 | 53 | 175 | 301 | a118 |
| NB 194-P7 0 | 36 | 74 | 176 | 302 | a119 |
| NB 194-P7 1 | 19 | 53 | 177 | 303 | a120 |
| NB 194-P7 2 | 19 | 53 | 178 | 304 | a121 |
| NB 194-P7 3 | 19 | 53 | 179 | 305 | a122 |
| NB 194-P7 4 | 19 | 53 | 180 | 306 | a123 |
| NB 194-P7 5 | 19 | 53 | 181 | 307 | a124 |
| NB 194-P7 6 | 19 | 53 | 182 | 308 | a125 |
| NB 194-P7 7 | 19 | 53 | 183 | 309 | a126 |
| NB194-P2 9-z 1 | 37 | 75 | 135 | 310 | a127 |
| NB194-P2 9-z2 | 37 | 75 | 135 | 311 | a128 |
| NB194-P2 9-z3 | 37 | 75 | 135 | 312 | a129 |
| NB194-P2 9-z4 | 37 | 75 | 135 | 313 | a130 |
| NB194-P2 9-z5 | 37 | 75 | 135 | 314 | a131 |
| NB194-P2 9-z6 | 37 | 75 | 135 | 315 | a132 |
| NB194-P2 9-z7 | 37 | 75 | 135 | 316 | a133 |
| NB194-P2 9-z8 | 37 | 75 | 135 | 317 | a134 |
| NB194-P2 9-z9 | 320 | 75 | 135 | 318 | a135 |
| NB194-P2 9-z 10 | 37 | 75 | 135 | 319 | a136 |

Preferably, the single domain antibody comprises CDR1, CDR2 and CDR3 selected from any one of the following groups: a14, a17, a40-a136, or preferably, the single domain antibody comprises CDR1, CDR2 and CDR3 selected from any one of the following groups: a14, a40-a44, or preferably, the single domain antibody comprises CDR1, CDR2 and CDR3 selected from any one of the following groups: a17, a45-a136.

In one or more embodiments, FR1 of the single domain antibody VHH can be selected from the FR1 of VHH of each antibody numbering in Table 1, FR2 of VHH can be selected from the FR2 of VHH of each antibody numbering in Table 1, FR3 of VHH can be selected from the FR3 of VHH of each antibody numbering in Table 1, and FR4 of VHH can be selected from the FR4 of VHH of each antibody numbering in Table 1.

In the preferred embodiment, the FR region of the single domain antibody is the FR region of any of the VHHs selected from SEQ ID NOs: 184-319. Further preferably, the CDRs of such antibodies are selected from the CDRs shown in any of the aforementioned groups a1 to a136. In one or more embodiments, the single domain antibody VHH is as shown in any one of SEQ ID NOs: 184-319. Preferably, the single domain antibody is as shown in any one of SEQ ID NOs: 197, 200, 223-319.

The PD-1 binding molecule described herein may be a monovalent or multivalent single domain antibody, a multispecific single domain antibody, a heavy chain antibody, or an antigen binding fragment thereof, an antibody, or an antigen binding fragment thereof, comprising one, two, or more anti-PD-1 single domain antibodies described herein. The heavy chain antibody also comprises a heavy chain constant region, such as the constant region of camelid heavy chain antibody or shark heavy chain antibody. Preferably, the heavy chain constant region is shown in SEQ ID NO: 321.

The description also comprises the antibody derivatives and analogues. "Derivatives" and "analogues" refer to polypeptides that basically maintain the same biological function or activity of the antibody of the present description. The derivatives or analogues of the present description may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

Without substantially affecting the activity of the antibody, those skilled in the art may change one or more (for example, 1, 2, 3, 4, 5, 6, 7, 8, 9 or 10 or more) amino acids to the sequence of the description to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or CDR regions of the variable region. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description.

The variant forms of the antibody described herein include: homologous sequence, conservative variant, allelic variant, natural mutant, induced mutant, protein encoded by DNA that can hybridize with the coding DNA of the antibody of the description under high or low strictness conditions, and polypeptide or protein obtained by using the antiserum of the antibody of the description.

In some embodiments, the sequence of the variant of the present description may have at least 95%, 96%, 97%, 98% or 99% identity with its source sequence. The sequence identity described in the description can be measured using sequence analysis software. For example, the computer program BLAST with default parameters, especially BLASTP or TBLASTN. The description also comprises those molecules with variable regions of antibody heavy chain with CDRs, if their CDRs have more than 90% homology (preferably more than 95%, more preferably more than 98%) with the CDRs identified here.

The antibody of the description can be prepared by conventional methods in the art, such as hybridoma technology well known in the art. The heavy chain antibody of the description can be prepared by conventional methods in the art, such as phage display technology well known in the art. Alternatively, the antibodies or heavy chain antibodies of the present description may be expressed in other cell lines. Suitable mammalian host cells can be transformed with sequences encoding the antibodies of the present description. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, Polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc. Particularly preferred cell lines are selected by determining which cell lines have high expression levels and produce antibodies with substantial PD-1 binding properties.

### Nucleic acid

The description also provides polynucleotides encoding the above antibody or fragments thereof. Polynucleotides encoding heavy chain variable region, light chain variable region, heavy chain, light chain and CDRs are provided. The polynucleotide of the description can be in the form of DNAs or RNAs. DNAs include cDNAs, genomic DNAs, or synthetic DNAs. DNAs can be single stranded DNAs or double stranded DNAs. DNAs can be coding or noncoding strand DNAs.

As those skilled in the art will understand, due to the degeneracy of the genetic code, an extremely large number of nucleic acids can be prepared, all of which encode the antibody of the description or antigen binding fragment thereof. Therefore, when a specific amino acid sequence has been identified, those skilled in the art can simply modify the sequence of one or more codons without changing the amino acid sequence of the encoded protein to produce any number of different nucleic acids. Therefore, the present description also relates to polynucleotides that hybridize with the above polynucleotide sequences and have at least 50%, preferably at least 70%, more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the antibody of the description or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein.

Once the relevant sequences are obtained, they can be obtained in large quantities by recombination. They are related sequences usually cloned into vectors, transferred into cells, and then isolated from the proliferated host cells by conventional methods. The biomolecules (nucleic acids, proteins, etc.) according to the present description include biomolecules in isolated form. At present, the DNA sequence encoding the protein (or fragment thereof, or derivative thereof) of the description can be obtained completely through chemical synthesis. This DNA sequence can then be introduced into various existing DNA molecules (or such as vectors) and cells known in the art. In addition, mutations can also be introduced into the protein sequence of the description through chemical synthesis.

Therefore, the present description also relates to nucleic acid constructs, such as expression vectors and recombinant vectors, comprising the above appropriate DNA sequence and the appropriate promoter or control sequence. These vectors can be used to transform appropriate host cells to enable them to express proteins. Vectors usually contain sequences for plasmid maintenance and for cloning and expressing exogenous nucleotide sequences. The sequences (collectively referred to as "flanking sequence" in some embodiments) generally comprises one or more of the following nucleotide sequences: promoter, one or more enhancer sequences, replication origin, transcription termination sequence, complete intronic sequence comprising donor and receptor splice sites, sequence encoding leader sequence for polypeptide secretion, ribosome binding site, polyadenylation sequence, a multi-linker region for inserting nucleic acids encoding antibodies to be expressed and an optional marker element. Exemplary vectors can be found in CN105154473A and CN111206043A, which are incorporated herein by reference in their entirety.

Host cells can be prokaryotic cells, such as bacterial cells; or lower eukaryotic cells, such as yeast cells; or higher eukaryotic cells, such as mammalian cells. Representative examples are: Escherichia coli, Streptomyces; bacterial cells of Salmonella typhimurium; fungal cells such as yeast; insect cells of Drosophila S2 or Sf9; animal cells of CHO, COS7, 293 cells, etc.

In some embodiments, host cells may be various functional cells well known in the art, such as various killer cells, including but not limited to cytokine induced killer cells (CIK), dendritic cell stimulated cytokine induced killer cells (DC-CIK), cytotoxic T lymphocytes (CTL), γδ T cells, natural killer cells (NK), tumor infiltrating lymphocytes (TIL), lymphokine activated killer cells (LAK), CD3AK cells (killer cells of anti-CD3 mAb), and CAR-T/TCR-T cells. In some embodiments, the killer cells are T cells or NK cells. Exemplary NK cells include, but are not limited to, primary NK cells, NK cell strains (such as NK92), and NKT cells. In some embodiments, the NK cells are primary NK cells. Exemplary T cells include, but are not limited to, peripheral blood T lymphocytes, cytotoxic T cells (CTLs), helper T cells, inhibitory/regulatory T cells, γδ T cells and mixed T cell populations of cytokine induced killer cells (CIK) and tumor infiltrating lymphocytes (TIL). In some embodiments, the T cells are peripheral blood T lymphocytes and TIL derived T cells.

In certain embodiments, the host cell expresses a chimeric antigen receptor and/or comprises a coding sequence that expresses a chimeric antigen receptor (CAR).

Transformation of host cells with recombinant DNA can be performed using conventional techniques known to those skilled in the art. When the host is a prokaryote such as Escherichia coli, competent cells that can absorb DNA can be harvested after the exponential growth period and treated with CaCl₂ method, the steps of which are well known in the art. Another method involves the use of MgCl₂. If necessary, the transformation can also be performed by electroporation. When the host is eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the description. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

### Use of treatment and pharmaceutical composition

By constructing a nanobody library, the inventor found, expressed, and purified 126 nanoantibodies and their variants that could bind PD-1. The binding of these antibodies to antigen and cells and drug safety were verified by affinity detection at protein level, affinity detection at cell level, PD-1 binding detection for tumor cells, epitope competition experiments and tissue cross-reaction.

All aspects of the antibodies described herein can be used to prepare medicaments to prevent or treat various conditions and diseases described herein, especially those related to PD-1 expressing cells. In some embodiments, the conditions and diseases are cancers, including but not limited to, melanoma, lung cancer, head and neck cancer, renal cell carcinoma, urothelial carcinoma, non-Hodgkin's lymphoma, and the like.

The pharmaceutical composition herein comprises the binding molecules described herein, as well as a pharmaceutically acceptable excipient, including but not limited to diluents, vehicles, solubilizers, emulsifiers, preservatives, and/or adjuvants. The excipients are preferably non-toxic to the recipient at the dose and concentration used. Such excipients include (but are not limited to): saline, buffer, glucose, water, glycerol, ethanol, and their combinations. In some embodiments, the pharmaceutical composition may contain substances for improving, maintaining, or retaining, for example, the pH, permeability, viscosity, clarity, color, isotonicity, odor, sterility, stability, dissolution or release rate, absorption, or permeation of the composition. These substances are known in the prior art. The optimal pharmaceutical composition can be determined according to the expected route of administration, mode of delivery, and required dose.

Pharmaceutical compositions for in vivo administration are usually provided in the form of sterile formulations. Sterilization is achieved by filtration through a sterile filter membrane. When the composition is lyophilized, this method can be used for sterilization before or after lyophilization and rehydration. The pharmaceutical composition of the present description may be selected for parenteral delivery. Compositions for parenteral administration may be in lyophilized form or stored in solution. For example, it is prepared by conventional methods with normal saline or aqueous solution comprising glucose and other adjuvants. Parenteral compositions are usually placed in containers with sterile access holes, such as intravenous solution strips or vials with plugs that can be pierced by subcutaneous injection needles. Alternatively, compositions may be selected for inhalation or delivery through the digestive tract, such as oral. The preparation of the pharmaceutically acceptable composition is within the art. Other pharmaceutical compositions will be apparent to those skilled in the art, including formulations comprising antibodies in sustained or controlled release delivery formulations. The techniques used to prepare a variety of other sustained or controllable delivery modes (such as liposome carriers, bioerodible particles or porous beads, and deposit injection) are also known to those skilled in the art.

Once the pharmaceutical composition is prepared, it is stored in sterile vials in the form of solution, suspension, gel, emulsion, solid, crystal or dehydrated or lyophilized powder. The formulation may be stored in ready to use form or rehydrated before administration (e.g., lyophilized). The description also provides a kit for generating a single dose administration unit. The kit of the description can each contain a first container with dried protein and a second container with aqueous formulation. In some embodiments of the present description, a kit comprising single-chamber and multi-chamber pre-filled syringes (e.g., liquid syringes and lyophilized syringes) are provided.

The present description also provides a method for treating a patient (especially a patient's PD-1 related disease) by administering a binding molecule or a pharmaceutical composition thereof according to any embodiment of the present description. Terms "patient", "subject", "individual" and "object" are used interchangeably herein, including any organism, preferably animals, more preferably mammals (such as rats, mice, dogs, cats, rabbits, etc.), and most preferably humans. "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a reduced rate of tumor metastasis or tumor growth). The treatment scheme for effectively treating patients can vary according to many factors, such as the patient's disease status, age, weight, and the ability of the therapy to stimulate the subject's anti-cancer response.

The therapeutically effective amount of the pharmaceutical composition comprising the binding molecule of the present description to be used will depend on, for example, the degree and the target of treatment. Those skilled in the art will understand that the appropriate dose level for treatment will vary in part depending on the molecule delivered, the indication, the route of administration, and the size (body weight, body surface or organ size) and/or condition (age and general health condition) of the patient. In some embodiments, clinicians may titrate the dose and change the route of administration to obtain the optimal therapeutic effect. For example, about 10 micrograms/kg body weight per day to about 50 mg/kg body weight.

The dosing frequency will depend on the pharmacokinetic parameters of the bound molecules in the formulation used. Clinicians typically administer the compositions until a dosage that achieves the desired effect. The composition may therefore be administered as a single dose, or over time as two or more doses (which may or may not contain the same amount of the desired molecule), or as a continuous infusion through an implanted device or catheter.

The route of administration of the pharmaceutical composition is according to known methods, such as oral, intravenous, intraperitoneal, intracerebral (intraparenchymal), intraventricular, intramuscular, intraocular, intra-arterial, portal vein or intralesional injection; by continuous release system or by implantable device.

### Diagnosis, detection, and kit

The binding molecule of the present description can be used in assays due to its high avidity with PD-1, such as binding assays to detect and/or quantify PD-1 expressed in tissues or cells. Binding molecules such as single domain antibodies can be used in further studies investigating the function of PD-1 in disease. The methods for detecting PD-1 are roughly as follows: obtaining cell and/or tissue samples; and detecting the level of PD-1 in the sample.

The PD-1 binding molecule of the description can be used for diagnostic purposes to detect, diagnose, or monitor PD-1 related diseases and/or conditions. The description provides the detection of the presence of PD-1 in a sample using a classical immunohistological method known to those skilled in the art. Detection of PD-1 can be performed *in vivo* or *in vitro.* Examples of methods suitable for detecting the presence of PD-1 include ELISA, FACS, RIA, and the like.

For diagnostic uses, binding molecules such as single domain antibodies are usually labeled with detectable labeling groups. Suitable labeling groups include (but are not limited to): radioisotopes or radionuclides (e.g., 3H, 14C, 15N, 35S, 90Y, 99Tc, 111In, 1251, 1311), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present description.

Another aspect of the present description provides a method for detecting the presence of a test molecule that competes with an antibody of the present description to bind PD-1. An example of one such assay would involve detecting the amount of free antibody in a solution comprising an amount of PD-1 in the presence or absence of the test molecule. An increase in the amount of free antibody (i.e., antibody that does not bind PD-1) will indicate that the test molecule can compete with the antibody for binding to PD-1. In one embodiment, the antibody is labeled with a labeling group. Alternatively, the test molecule is labeled, and the amount of free test molecule is monitored in the presence or absence of the antibody.

The description also provides a detection kit for detecting PD-1 level. The kit comprises an antibody that recognizes PD-1 protein, a lysis medium for dissolving samples, and general reagents and buffers required for detection, such as various buffers, detection markers, detection substrates, etc. The detection kit can be an in vitro diagnostic device.

The description specifically comprises an embodiment shown in any one of the following:
1. A PD-1 binding molecule, comprising an anti-PD-1 single domain antibody, wherein the complementarity determining region CDRs of the single domain antibody comprise CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3, wherein,
   SEQ ID NO: 1 is GX₁X₂X₃X₄X₅X₆X₇, wherein X₁ is D, F, G, H, L or R, X₂ is P, S or T, X₃ is A, F, I, L, N, R, S or V, X₄ is D, F, G, L, M, N or S, X₅ is A, D, F, G, I, N, R, S or T, X₆ is D, F, H, I, L, S, V, W or Y, X₇ is A, D, E, G, N, P, S or T; preferably, SEQ ID NO: 1 is GX₁X₂X₃X₄X₅X₆X₇, wherein X₁ is D, F, G, H, L or R, X₂ is P, S or T, X₃ is F or S, X₄ is F or S, X₅ is G, I or T, X₆ is S or Y, X₇ is A or D,
   SEQ ID NO: 2 is X₁X₂X₃X₄X₅X₆X₇X₈X₉, wherein X₁ is I, L, S, T or V, X₂ is A, N, S or T, X₃ is F, G, I, K, L, M, N, Q, R, S, T, W or Y, X₄ is A, D, G, H, N, R, SorT, X₅ is A, D, G, N, R, S or null, X₆ is G or R, SorT, X₇ is D, E, I, L, M, N, R, S, T or V, X₈ is A, K, M, Q or T, X₉ is A or null; preferably, SEQ ID NO :2 is X₁X₂X₃X₄XₛGX₇TX₉, where X₁ is I or V, X₂ is N, S or T, X₃ is F, L or N, X₄ is A, G, S or T, X₅ is R or null, and X₇ is D, N , I or T, X₉ is null,
   SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, E, G, N, R, S, T or V, X₂ is A, G, I, K, L, P, R, T or V, X₃ is A, D, E, G, K, N, P, R, S, V or Y, X₄ is A, C, D, E, G, I, K, L, R, S, T, V or Y, X₅ is A, C, D, F, G, H, I, K, L, N, P, Q, R, S, W or Y, X₆ is A, C, D, E, G, I, K, L, M, P, Q, R, S or Y, X₇ is A, C, D, F, G, H, I, N, P, R, S, T, V or Y, X₈ is A, C, D, E, G, I, R, S, T, V, W, Y or null, X₉ is A, D, E, F, G, I, L, P, R, S, V, W, Y or null, X₁₀ is C, D, F, G, K, L, N, P, R, S, T, V, W, Y or null, X₁₁ is D, F, G, H, I, K, L, N, P, S, T, V, Y or null, X₁₂ is A, D, G, H, I, L, M, P, R, S, T, V, Y or null, X₁₃ is C, D, E, H, P, R, S, T, V, Y or null, X₁₄ is A, D, E, F, H, I, L, P, R, T, Y or null, X₁₅ is A, D, E, G, N, Q, R, S, T, V, Y or null, X₁₆ is A, D, E, F, G, H, M, P, S, V , Y or null, X₁₇ is A, D, E, H, N, Q, R, T, Y or null, X₁₈ is D, E, R, S, Y or null, X₁₉ is N, S, Y or null, X₂₀ is D, E, Y or null, X₂₁ is E, N, Y or null, X₂₂ is Y or null; preferably, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₄ is E, G, N or V, X₂ is A, G, I, L or V, X₃ is A, D, E, G, N, V or Y, X₄ is A, G, I, K, R or S, X₅ is C, I, L, P, Q, R or W, X₆ is E, K, P, Q or R, X₇ is C, D, H, N, P or Y, X₈ is E, G, R, V or W, X₉ is F, G, L, S, V or W, X₁₀ is C, D, G, L, R, S or V, X₁₁ is F, G, I, L, N, S, T or V, X₁₂ is H, L, P, R, S, T or Y, X₁₃ is D, H, P, S, T or V, X₁₄ is E, F, H, I, L, P or R, X₁₅ is A, S or V, X₁₆ is A, D, E, G, H or V, X₁₇ is H, N, Q, R or Y, X₁₈-X₂₂ is null.
2. The PD-1 binding molecule according to Item 1, wherein CDR1 of the single domain antibody comprises the sequence shown in any one of SEQ ID NOs: 4-39 and 320, CDR2 comprises the sequence shown in any one of SEQ ID NOs: 40-75, and CDR3 comprises the sequence shown in any one of SEQ ID NOs: 76-183,
   preferably, the single domain antibody comprises CDR1, CDR2 and CDR3 shown in any one of groups a1 to a136 in Table 1.
3. The PD-1 binding molecule according to Item 1 or 2, wherein,
   the FR region of the single domain antibody comprises the FR region of any VHH selected from SEQ ID NOs: 184-319, and/or
   the single domain antibody VHH is as shown in any one of SEQ ID NOs: 184-319, and/or
   the PD-1 binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.
4. The PD-1 binding molecule according to Item 3, wherein the PD-1 binding molecule is a heavy chain antibody, which further comprises a heavy chain constant region,
   preferably, the heavy chain constant region comprises the sequence shown in SEQ ID NO: 321.
5. A polynucleotide, wherein the polynucleotide comprises a sequence selected from:
   (1) a coding sequence of the PD-1 binding molecule according to any one of Items 1-4;
   (2) a complementary sequence of (1);
   (3) a 5-50bp fragment of any sequence of (1) or (2).
6. A nucleic acid construct, wherein the nucleic acid construct comprises the polynucleotide according to Item 5,
   preferably, the nucleic acid construct is a recombinant vector or expression vector.
7. A Phage comprising the PD-1 binding molecules according to any one of Items 1-4,
   preferably, the PD-1 binding molecule is displayed on the surface of the phage.
8. A host cell, wherein the host cell:
   (1) expresses and/or secrets the PD-1 binding molecules according to any one of Items 1-4; and/or
   (2) comprises the polynucleotide according to Item 5; and/or
   (3) comprises the nucleic acid construct according to Item 6.
9. A method for producing PD-1 binding molecules, comprising: culturing the host cell according to Item 8 under conditions suitable for producing the PD-1 binding molecules, and optionally purifying the PD-1 binding molecules from the culture.
10. A pharmaceutical composition comprising the PD-1 binding molecule according to any one of Items 1-4, the polynucleotide according to Item 5, the nucleic acid construct according to Item 6, the phage according to Item 7 or the host cell according to Item 8, and a pharmaceutically acceptable excipient,
   preferably, the pharmaceutical composition is used for treating cancer,
11. Use of the PD-1 binding molecule according to any one of Items 1-4 in the preparation of a medicament for the prevention or treatment of a cancer.
12. A kit for detecting PD-1, for use in evaluating the therapeutic effect of a medicament or diagnose cancer, wherein the kit comprises the PD-1 binding molecule according to any one of Items 1-4, the polynucleotide according to Item 5, the nucleic acid construct according to Item 6, the phage according to Item 7 or the host cell according to Item 8,
   preferably, the kit further comprises a reagent for detecting the binding of PD-1 to a single domain antibody, an antibody, or an antigen binding fragment thereof,
   more preferably, the reagent is a reagent that detects the binding by enzyme-linked immunosorbent assay.
13. A non diagnostic method for detecting the presence of PD-1 in a sample, wherein the method comprises: incubating a PD-1 binding molecule according to any one of Items 1-4 with the sample, and detecting the binding of PD-1 to a single domain antibody, antibody, or antigen binding fragment thereof, thereby determining the presence of PD-1 in the sample.
14. Use of the PD-1 binding molecule according to any one of Items 1-4 in the preparation of a kit for detecting PD-1 in samples, evaluating the therapeutic effect of a medicament, or diagnosing a cancer.

The present invention will be described below in the form of specific examples. It should be understood that these examples are merely illustrative and are not intended to limit the scope of the present description. Unless otherwise specified, the methods and materials used in the examples are conventional materials and methods in the art.

### Example

### Example 1. Immunization of alpaca

### 1.1 Preparation of immunogen:

The sequence of PD-1 protein was obtained from NCBI, and was fused with the sequence of human IgG Fc fragment, and the eukaryotic expression vector of pCDNA3.4 (Thermo) plasmid was synthesized and constructed by Nanjing Genscript Company. The synthesized plasmid was expressed using ExpiCHO^{™} (Thermo Fisher) expression system. After expression, 5 ml of protein A pre-packed column (GE) was used for one-step affinity purification, and the purified sample was replaced into PBS buffer. After the purity was identified by SDS-PAGE electrophoresis gel and HPLC, and the activity was identified by ELISA, the sample was split and frozen in -80°C refrigerator for subsequent immunization.

### 1.2 Immunization of Alpaca:

For first immunization, 400 µg of antigen (PD-1.hFc) was mixed with the adjuvant (GERBU FAMA). Alpaca was immunized subcutaneously at four sites on the back, and the amount of each site was 1 mL. For the second to seventh immunization, 200 µg of antigen was used. Alpacas were immunized by subcutaneous injection at four sites on the back, and the amount of immunization at each site was 1 mL. The interval between immunizations was one week.

### 1.3 Detection of immune serum titer:

### 1.3.1 Detection of titer at protein level

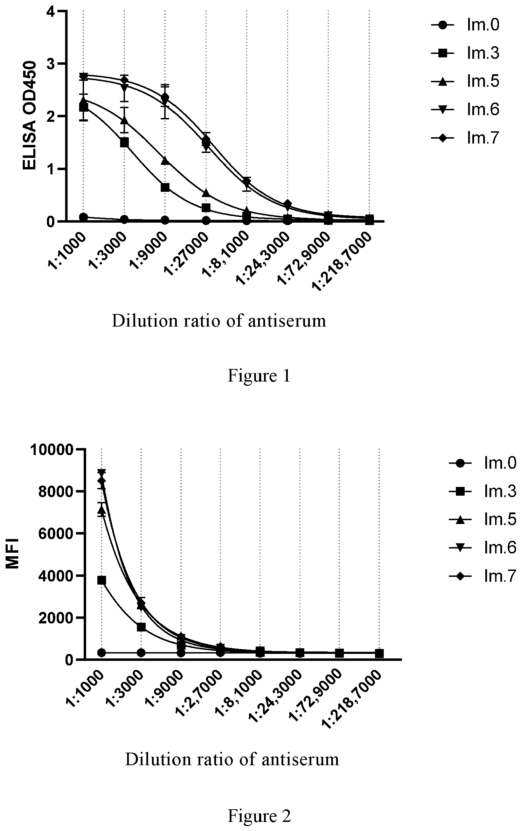

PD-1.His antigen was coated overnight at 4 degrees. After blocking and washing, the gradient diluted serum was added to the ELISA plate for incubation, and then incubated with the anti-llama (anti-Alpaca) IgG HRP (Abcam) antibody. After washing, TMB chromogenic solution was added for development, and the reaction was terminated with 2 M HCl. Then the absorbance value at OD450 nm was detected with a microplate reader. As shown in Figure 1, the titer of Alpaca reached a higher level (>81000) after seven immunizations.

### 1.3.2 Detection of titer at cell level

PD-1 transfected CHO-K1 cells were plated in 96 well plates with a cell volume of 3 × 10⁵ cells/well. The cells were then incubated with 3-fold gradient diluted serum. After incubation and washing, anti-llama IgG PE (Jackson) antibody was added for incubation. After washing, the cells were resuspended with PBS, and then the fluorescence intensity (MFI) was detected by flow cytometry (Beckman). After seven immunizations, the titer of alpaca at the cellular level reached 9000. The results were shown in Figure 2.

### Example 2. Construction and screening of nanobody immune library for PD-1

(1) After seven immunizations, 100 mL of camelid peripheral blood lymphocytes were extracted, and total RNA was extracted. RNA was extracted according to the instructions of RNAiso reagent of Takara.
(2) The first strand of cDNA was synthesized with RNA as template and oligo dT as primer according to the instructions of reverse transcriptase of Takara.
(3) The variable region coding gene of heavy chain antibody was obtained by nested PCR using PrimeSTAR high fidelity DNA polymerase. The variable region fragment of the heavy chain antibody was amplified by nested PCR:
First round of PCR:
   Upstream primer: GTCCTGGCTGCTCTTCTACAAGGC
   Downstream primer: GGTACGTGCTGTTGAACTGTTCC
   The fragment between the heavy chain antibody guide peptide and antibody CH2 was amplified, annealed at 55°C for 30 cycles; a DNA fragment of about 600 bp was recovered as a template for the second round of PCR.
Second round of PCR:
   Upstream primer: GATGTGCAGCTGCAGGAGTCTGGRGGAGG
   Downstream primer: GGACTAGTGCGGCCGCTGGAGACGGTGACCTGGGT
      The fragment (long fragment and short fragment) between the FR1 region and the long and short hinge regions of the heavy chain antibody was amplified, annealed at 55°C for 30 cycles, and the target fragment was recovered. The result showed that the size of the fragment was about 500 bp, that is, the nanobody gene electrophoresis band was about 500 bp.

(4) The phage pME207 and PCR amplification products were digested with Sfi I and Not I (NEB), respectively. After recovery and quantification, the two fragments were ligated with T4 DNA ligase (Takara) at a molar ratio of 1:3 and ligated overnight at 16°C.
(5) After ethanol precipitation, the ligated product was dissolved in 100 µL sterile water and electroporated into Escherichia coli TG1 in ten times. 100µL of the bacterial solution was taken after electric shock and culture, diluted by multiple ratio, coated on an ampicillin LB culture plate, the storage capacity was calculated, and the rest was coated with ampicillin 2×YT culture plate, at 37°C, invertly cultured for 13-16 h. After scraping and washing the colonies on the culture plate with 10 ml, 2×YT medium, 25% glycerol at the final concentration was added, split, and stored at -80°C for further use. The size of the storage capacity is 4.3 × 10⁹. To detect the insertion rate of the library, 48 clones were randomly selected for colony PCR, and the results showed that the insertion rate had reached more than 90%.
(6) According to the calculated library capacity results, viable cells with 10 times the library capacity were seeded in 200ml of 2×YT (comprising 2% glucose, 100 µg/ml ampicillin), cultured at 37°C for 200 r/min until the OD600 reached 0.5, auxiliary phage was added according to the multiplicity of infection of 20:1, and left for 30 min at 37°C, 200 r/min for 30 min. The culture was centrifuged, and the pellet was resuspended with 200 ml of 2×YT (comprising 100 µg/ml ampicillin and 50 µg/ml kanamycin), incubated overnight at 37°C, 250 r/min, centrifuged at 8000rpm to obtain the supernatant, added with 5×PEG/NaCl solution, placed on ice for 60 min, centrifuged at 8000rpm for 30 min. The pellet was resuspended in 5 ml of PBS to obtain the single domain heavy chain antibody (VHH) immune library against PD-1, and 10 µL was taken to determine the titer, and the rest were split at -80°C for storage.
(7) PD-1 was coated on an ELISA plate at 5 µg/ml, 100 µl per well, and placed overnight at 4 °C. At the same time, a negative control was set up. The next day, 200 µL, 3%BSA were added to the five wells and blocked for 2 hours at room temperature. Two hours later, they were washed three times with PBST (comprising 0.05% Tween 20 in PBS). After the plate was washed, 100 µL of phage pre-blocked with 5% skim milk (2-3 × 10¹¹ tfu immunized camelid nanobody phage display gene library) was added, left for 1.5 hours at room temperature, and then the supernatant after negative screening was transferred to the target antigen coated well for 1.5 hours at room temperature. It was washed with PBST (comprising 0.05% Tween 20 in PBS) for 12 times to wash out the unbound phage. The phage specifically bound to PD-1 was dissociated with Glycine (SIGMA), and the eluted phage was neutralized by Tris (Invitrogen, 1 M, pH 8.0) and infected with TG1 in logarithmic phase. After propagation and expansion, the next round of "adsorption-elution" was carried out. Finally, TG1 was impregnated with the eluted phage, and the expression of nanobody was induced by IPTG (Thermo). The supernatant expressing TG1 was taken for ELISA binding detection and PD-L1.hFc blocking detection. The secondary antibody used for ELISA binding detection is anti-c-myc Antibody HRP (Bethyl), and the secondary antibody used for blocking detection of PD-L1 binding is Goat anti-Human IgG-Fc Fragment Antibody HRP (Bethyl).
(8) According to sequence analysis, a total of 39 nanobodies that can bind to PD-1 protein were obtained, as shown in the following table.

| Clone No. | ELISA (OD450) | | |
|---|---|---|---|
| | Clone binding with PD-1 | PD-L1 binding with PD-1 | Negative control |
| NB139 | 3.122 | 2.394 | 0.026 |
| NB147 | 2.627 | 0.994 | 0.027 |
| NB148 | 2.841 | 2.563 | 0.026 |
| NB153 | 3.293 | 2.052 | 0.025 |
| NB154 | 2.608 | 2.281 | 0.026 |
| NB159 | 1.837 | 2.104 | 0.065 |
| NB 164 | 2.841 | 2.001 | 0.039 |
| NB 166 | 3.388 | 2.293 | 0.026 |
| NB 169 | 2.895 | 2.128 | 0.026 |
| NB170 | 2.701 | 2.015 | 0.026 |
| NB171 | 2.781 | 0.715 | 0.026 |
| NB172 | 3.052 | 2.168 | 0.027 |
| NB 173 | 2.974 | 0.794 | 0.027 |
| NB182 | 3.081 | 0.213 | 0.028 |
| NB189 | 2.968 | 2.337 | 0.035 |
| NB 192 | 2.647 | 2.240 | 0.026 |
| NB 194 | 2.835 | 0.152 | 0.025 |
| NB195 | 3.131 | 2.237 | 0.027 |
| NB 196 | 3.387 | 2.491 | 0.026 |
| NB 197 | 2.659 | 2.274 | 0.025 |
| NB200 | 2.305 | 2.477 | 0.025 |
| NB202 | 3.040 | 0.723 | 0.025 |
| NB205 | 2.248 | 2.204 | 0.027 |
| NB209 | 3.219 | 2.041 | 0.026 |
| NB211 | 2.711 | 2.211 | 0.026 |
| NB215 | 3.289 | 1.275 | 0.027 |
| NB216 | 2.746 | 2.451 | 0.026 |
| NB218 | 2.615 | 2.318 | 0.026 |
| NB220 | 2.378 | 2.206 | 0.026 |
| NB221 | 2.623 | 2.250 | 0.026 |
| NB222 | 3.128 | 2.227 | 0.027 |
| NB223 | 1.298 | 2.254 | 0.027 |
| NB532 | 1.358 | 2.193 | 0.026 |
| NB548 | 2.127 | 2.429 | 0.026 |
| NB733 | 1.033 | 2.471 | 0.026 |
| NB734 | 2.173 | 1.421 | 0.026 |
| NB735 | 1.815 | 2.437 | 0.026 |
| NB754 | 2.036 | 2.371 | 0.026 |
| NB755 | 1.704 | 2.204 | 0.025 |

### Example 3. Expression and purification of candidate antibodies

The nanoantibodies were constructed on pCDNA3.4-IgG4 vector, and then expressed by ExpiCHO^{™} (Thermo Fisher) expression system. After one week of expression, the supernatant was collected for protein A (GE) purification. Then Nanodrop was used to detect the protein quality, and HPLC was used to detect the protein purity. The purity and yield of the obtained protein met the requirement of subsequent tests. Keytruda and Opdivo IgG4 antibodies were prepared based on the sequences provided at https://www.drugbank.ca/.

### Example 4. Characterization of candidate antibodies

Detection of affinity at protein level: the binding kinetics and affinity of antibody to human PD-1.Fc antigen were determined using surface plasmon resonance (SPR). The purified antibody was flowed through the sensor chips pre-immobilized with protein A, and the antibody was captured by protein A. Then five different concentrations of PD-1. Fc protein were used as the mobile phase, and the binding time and dissociation time were 30min and 60min, respectively. The binding rate (ka), dissociation rate (kd), and equilibrium constant (kd) were analyzed using Biacore Evaluation Software 2.0 (GE). The marketed PD-1 antibody medicaments Keytruda and Opdivo were selected as controls. The results are shown in the Table below.

| Sample | ka (1/Ms) | kd (1/s) | KD (M) |
|---|---|---|---|
| NB147 | 5.58E+04 | 1.53E-04 | 2.75E-09 |
| NB171 | 4.97E+04 | 3.09E-04 | 6.21E-09 |
| NB 173 | 2.15E+03 | 1.79E-04 | 8.31E-08 |
| NB182 | 7.38E+03 | 6.40E-05 | 8.67E-09 |
| NB202 | 2.06E+05 | 6.77E-05 | 3.28E-10 |
| NB211 | 2.26E+06 | 3.00E-03 | 1.33E-09 |
| NB215 | 4.90E+04 | 5.08E-04 | 1.04E-08 |
| NB223 | 1.35E+05 | 8.63E-04 | 6.39E-09 |
| NB 194 | 1.13E+06 | 7.62E-05 | 5.62E-10 |
| NB220 | 1.48E+06 | 3.23E-04 | 2.19E-10 |
| NB222 | 2.05E+06 | 3.06E-04 | 1.49E-10 |
| NB532 | 1.31E+05 | 9.20E-04 | 7.03E-09 |
| NB734 | 2.36E+05 | 6.88E-05 | 2.92E-10 |
| Other antibody | 2.31E+4 to 5.01E+6 | 8.11E-03 to 7.95E-5 | 2.74E-8 to 6.32E-10 |
| Keytruda | 1.01E+06 | 3.33E-04 | 3.29E-10 |
| Opdivo | 7.08E+05 | 9.48E-05 | 1.34E-10 |

### Example 5: Different Species PD-1 Binding

PD-1 protein (ACROBiosystems) of different species (human, cynomolgus monkey, murine) were coated on ELISA plates at a concentration of 1 µg/ml, and placed at 4°C overnight. After blocking and washing, the diluted antibodies were added to the ELISA plate and incubated at room temperature for 2 hours. After washing, Goat anti-Human IgG-Fc Fragment Antibody HRP(Bethyl) secondary antibody was added and incubated at room temperature for 1 hour. After washing, TMB chromogenic solution was added, and then the reaction was terminated with 2M HCl, and the OD450 value was read using a microplate reader.

The experimental results are shown in the table below. The candidate antibodies, Keytruda and Opdivo all bind to human and cynomolgus monkey PD-1, but not to murine PD-1 protein.

| Sample | ELISA (OD) | | | | | | | | |
|---|---|---|---|---|---|---|---|---|---|
| | Coated with human PD-1 protein | | | Coated with cynomolgus monkey PD-1 protein | | | Coated with murine PD-1 protein | | |
| NB182, 0.1µg | 2.174 | 2.130 | 2.100 | 2.070 | 2.075 | 1.949 | 0.068 | 0.071 | 0.061 |
| NB194, 0.1µg | 2.220 | 1.947 | 2.108 | 2.225 | 2.170 | 2.097 | 0.024 | 0.022 | 0.024 |
| Other antibody,0.1µg | 0.723-2.231 | | | 0.533-2.179 | | | 0.020-0.081 | | |
| Keytruda, 0.1µg | 2.254 | 2.336 | 2.184 | 2.222 | 2.340 | 2.319 | 0.079 | 0.088 | 0.090 |
| Opdivo, 0.1µg | 2.112 | 2.144 | 2.248 | 1.888 | 1.795 | 1.834 | 0.024 | 0.023 | 0.023 |

### Example 6: CD28 family protein binding assay

The CD28 family proteins (CD28, CTLA-4, ICOS and PD-1) (ACROBiosystems) were coated on the microtiter plate respectively at a concentration of 1 µg/ml and placed at 4°C overnight. After blocking and washing, the diluted antibodies were added to the ELISA plate and incubated at room temperature for 2 hours. After washing, Goat anti-Human IgG-Fc Fragment Antibody HRP(Bethyl) secondary antibody was added and incubated at room temperature for 1 hour. After washing, TMB chromogenic solution was added, and then the reaction was terminated with 2M HCl, and the OD450 value was read using a microplate reader.

The experimental results are shown in the table below. The candidate antibodies, Keytruda and Opdivo only bind to the PD-1 protein, and do not bind to other proteins of the CD28 family.

| Sample | ELISA (OD) | | | | | | | | | | | |
|---|---|---|---|---|---|---|---|---|---|---|---|---|
| | CTLA-4 | | | ICOS | | | CD28 | | | PD-1 | | |
| NB 182 | 0.191 | 0.186 | 0.185 | 0.110 | 0.134 | 0.111 | 0.053 | 0.049 | 0.047 | 2.055 | 2.093 | 2.287 |
| NB 194 | 0.033 | 0.028 | 0.026 | 0.022 | 0.021 | 0.023 | 0.022 | 0.022 | 0.020 | 2.034 | 1.957 | 2.272 |
| Other antibody | 0.011-0.108 | | | 0.017-0.141 | | | 0.019-0.046 | | | 0.821-2.346 | | |
| Keytruda | 0.090 | 0.086 | 0.089 | 0.125 | 0.107 | 0.131 | 0.066 | 0.054 | 0.053 | 2.118 | 2.209 | 2.452 |
| Opdivo | 0.024 | 0.022 | 0.019 | 0.023 | 0.021 | 0.022 | 0.020 | 0.021 | 0.023 | 2.051 | 1.999 | 2.350 |

### Example 7. Epitope competition experiment

The instrument Biacore T200 (GE), detection temperature of 25 °C, buffer of HBS-EP+(10 mM HEPES, 150 mM NaCl, 3 mM EDTA and 0.05% v/v Surfactant P20, GE) at a flow rate of 30 ul/min was used. PD-1-His was dissolved in acetate buffer, pH4.0 (Biacore Amine Coupling Kit, GE), to a final concentration of 10 µg/ml. According to the product instructions of amino coupling kit, PD-1-His was fixed to CM5 chip (GE) channel 2 (FC-2), about 21 RU. Channel 1 (FC-1) was used as blank control. Antibodies were diluted with 1 × HBS-EP+ to the target concentration, respectively, one antibody was injected to the above PD-1 fixed chips FC-1 and FC-2 to the saturation level, and then the other antibody was injected to the saturation level to evaluate the competition relationship between the two antibodies. After the second antibody injection, the complex was dissociated for 400s. Finally, 50 mM NaOH was injected for 15s to regenerate the surface of the chip. Biacore Evaluation Software 2.0 (GE) was used for data processing, dual reference subtraction was used for sensorgrams, and FC2-1 signals were recorded.

The experimental results are shown in the table below. The epitopes bound by NB194 and NB182 antibodies partially overlapped with those of Keytruda and Opdivo. The results of the remaining antibodies were similar to those of the NB194 and NB182 antibodies, both competing with Keytruda and Opdivo for the epitope moiety.

| The First antibody | The Second antibody | | | |
|---|---|---|---|---|
| | NB 194 | NB182 | Keytruda | Opdivo |
| NB 194 | Complete competition | Partial competition | Partial competition | Partial competition |
| NB182 | Complete competition | Complete competition | NA | NA |
| Keytruda | Complete competition | Partial competition | Complete competition | Partial competition |
| Opdivo | Complete competition | Partial competition | Partial competition | Complete competition |

### Example 8: Antibody functional activity test

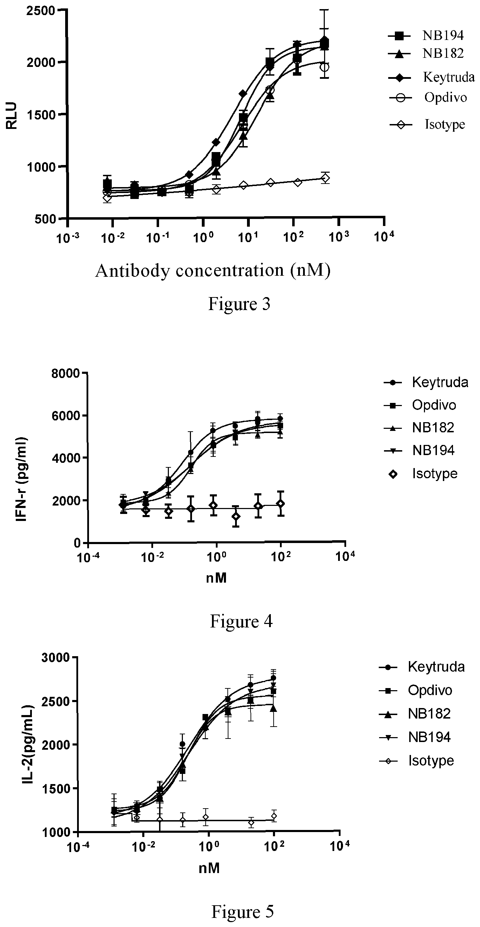

1)Reporter gene experiments. First, the GS/C2-PDL1 (GenScript) target cells were adjusted to a density of 5^{∗}10⁵ cells/mL with antibiotic-free medium, and was plated in a 384-well plate at 20 µL per well for 16-20 hours. The antibody was serially diluted with RMPI1640+10% FBS buffer, and the density of GS-J2/PD1 (GenScript) effector cells was adjusted to 2^{∗}10⁶ cells/mL with antibiotic-free medium; the old medium in the 384-well plate was removed, and 20 µL of antibody and 20 µL of GS-J2/PD1 effector cells were added to each well and cultured for 6 h. Finally, 40 µL of One-Glo substrate was added to each well to react for 10 minutes and then detected by microplate reader. As shown in Figure 3, the antibodies had good functional activity in the Reporter Gene Assay functional detection method, with an EC50 of 16.3 nM and 7.0 nM for NB182 and NB194, and an EC50 of 3.3 nM and 7.3 nM for Keytruda and Opdivo. The remaining antibodies showed EC50s between 1.7 and 75 nM.
2) Mixed lymphocyte reaction. 100 ml of peripheral blood was drawn from healthy people for PBMC isolation, and the obtained PBMC was then used for monocyte isolation. The obtained monocyte was induced into mature dendritic cell DC within nine days. On Day 9, DCs were tested for quality by flow cytometry. After meeting the standards for induction of maturation, 100 ml of human peripheral blood was obtained again for PBMC isolation, and then CD4+ T cells were isolated from PBMC, a ratio of 5:1 of the E:T ratio was used to incubate effector cells and stimulated cells, and the samples to be tested were added at 8 concentrations, each 3 replicate wells, with 10-fold dilution, and the initial concentration was 10-50ug/ml. Cells were incubated for 3 days. The supernatant of cell culture was collected, and the amount of IL-2 and IFN-γ released was detected.

The test results are shown in Figure 4 and Figure 5. Within a certain range, NB182 and NB194 can promote T cells to secrete IL-2 and IFN-γ in a dose-dependent manner, and exhibit the same functional effects as Keytruda and Opdivo. The results of the remaining antibodies were similar to NB182 and NB194, all of which could promote T cells to secrete IL-2 and IFN-γ in a dose-dependent manner: EC50 of IL-2 was between 0.115nM and 3.278nM, and EC50 of IFN-γ was between 0.078nM and 1.102nM.

### Example 9: Antibody specificity verification

### 1) Membrane protein cross-reaction

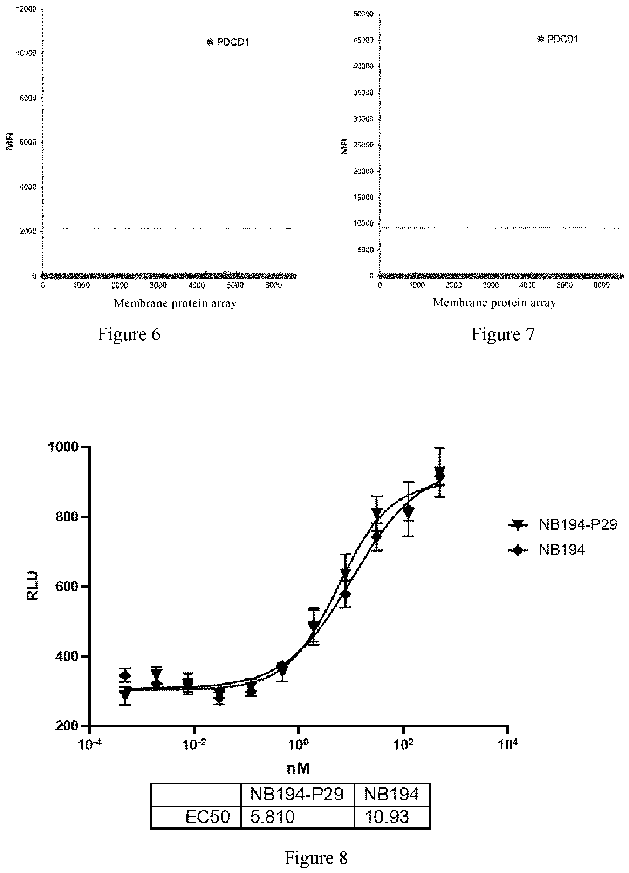

Membrane protein cross-reaction was performed using the membrane protein array screening platform (Membrane Proteome Array, MPA) developed by Integral molecular, United States. 5300 different human membrane proteins were displayed on the cell surface by transfecting HEK293 cells, and the binding signals of the antibodies on these proteins were detected by FACS to evaluate the specificity of the antibodies to be detected. The results of MPA screening (Fig. 6 and Fig. 7) showed that NB182 and NB194 antibodies only bound to human PD-1 protein and did not bind to more than 5000 other proteins. Except for the NB171 antibody, the other antibodies only bind to human PD-1 protein.

### 2) tissue cross-reaction

34 tissues were selected for frozen section, dried at room temperature, and fixed with acetone. Blocking was performed using Reagent A and Reagent B of IHC Biotin Block Kit (Sangon, E674001). Biotin labeled antibody samples were incubated for 30min, and horseradish peroxidase labeled streptavidin (Abeam, ab7403) was added for incubation for 15min after washing. DAB development, hematoxylin counterstaining, neutral plastic sealing, natural air drying, and then microscopic examination were performed. The positive control was Anti-PD-1 antibody (abeam), and the negative control was the IgG4 isotype control.

NB182 and NB194 antibodies specifically bind to normal human lymphocytes, including lymph nodes, spleen, and thymus. Except for the NB171 antibody, the other antibodies only specifically bind to normal human lymphocytes.

| No. | Organ | NB182 | **NB194** | isotype control |
|---|---|---|---|---|
| 1 | Adrenal gland | - | - | - |
| 2 | Breast | - | - | - |
| 3 | Eye | - | - | - |
| 4 | Kidney | - | - | - |
| 5 | Pancreas | - | - | - |
| 6 | spinal cord | - | - | - |
| 7 | Thyroid | - | - | - |
| 8 | Aorta | - | - | - |
| 9 | Cerebellum | - | - | - |
| 10 | Esophagus | - | - | - |
| 11 | Liver | - | | - |
| 12 | Pituitary | - | - | - |
| 13 | Spleen | lymphocyte-lik e positive staining | lymphocyte-lik e positive staining | - |
| 14 | Ureter | - | - | - |
| 15 | Bladder | - | - | - |
| 16 | Brain | - | - | - |
| 17 | Oviduct | - | - | - |
| 18 | Lung | - | - | - |
| 19 | Placenta | - | - | - |
| 20 | Skeletal muscle | - | - | - |
| 21 | Cervix | - | - | - |
| 22 | Blood cells | - | - | - |
| 23 | Colon | - | - | - |
| 24 | Stomach | - | - | - |
| 25 | Lymph node | lymphocyte-lik e positive staining | lymphocyte-lik e positive staining | - |
| 26 | Prostate | - | - | - |
| 27 | Testis | - | - | - |
| 28 | Endometrial cancer | - | - | - |
| 29 | Marrow | - | - | - |
| 30 | Duodenum | - | - | - |
| 31 | Heart | - | - | - |
| 32 | Ovary | - | - | - |
| 33 | Skin | - | - | - |
| 34 | Thymus | lymphocyte-lik e positive staining | lymphocyte-lik e positive staining | - |

### Example 10: Antibody humanization

Referring to the method of humanizing nanoantibodies (J. Biol. Chem. 2009; 284: 3273-3284), NB182 and NB194 was humanized using the method of CDR region transplantation. In IgBLAST (http://www.ncbi.nlm.nih.gov/igblast/), germline with high homology with NB182 and NB194 was selected as the template in the database. Biacore was used to detect the affinity of the antibody before and after humanization. The purified antibody was flowed through the sensor chip pre-fixed with protein A, and the antibody was captured by protein A. Then five different concentrations of PD-1.His protein were used as the mobile phase, and the association time and dissociation time were 30 min and 60 min, respectively. The binding rate (ka), dissociation rate (kd), and equilibrium constant (kd) were analyzed using Biacore Evaluation Software 2.0 (GE). The two humanized molecules NB182-z1 and NB182-z2 had the same affinity with NB182, and NB182-z3, NB182-z4, NB182-z5 had similar affinity with NB182. The two humanized molecules NB194-z4 and NB194-z5 had the same affinity with NB194, and NB194-z1, NB194-z2, NB194-z3 had similar affinity with NB194.

| Sample | ka(1/Ms) | kd (1/s) | KD (M) |
|---|---|---|---|
| NB182 | 7.38E+03 | 6.40E-05 | 8.67E-09 |
| NB182-z1 | 9.22E+03 | 7.61E-05 | 8.25E-09 |
| NB 182-z2 | 5.60E+03 | 4.59E-05 | 8.20E-09 |
| NB 182-z3 | 6.00E+03 | 7.1 1E-05 | 1.18E-08 |
| NB 182-z4 | 6.48E+03 | 9.73E-05 | 1.50E-08 |
| NB 182-z5 | 6.46E+03 | 9.07E-05 | 1.40E-08 |
| NB 194 | 2.12E+05 | 1.05E-04 | 4.94E-10 |
| NB194-z1 | 1.28E+05 | 1.07E-04 | 8.37E-10 |
| NB194-z2 | 9.76E+04 | 9.88E-05 | 1.01E-09 |
| NB 194-z3 | 1.41E+05 | 1.39E-04 | 9.87E-09 |
| NB194-z4 | 1.33E+05 | 7.79E-05 | 5.86E-10 |
| NB194-z5 | 1.33E+05 | 7.92E-05 | 5.98E-10 |

### Example 11: Mutant Antibodies

Point random mutation of NB194 antibody was performed by GeneMorph II kit (Agilent, 200550), and then a phage antibody library was constructed. After screening, 77 mutant antibodies (NB194-P1 to NB194-P77) were obtained. The affinity and functional activity of these antibodies were verified to be similar to those of the NB194 antibody. As shown in the table below and Figure 8, the NB194-P29 mutation had the highest affinity, with a 5-fold increase in affinity and improved functional activity compared to NB194.

| Sample | ka (1/Ms) | kd (1/s) | KD (M) |
|---|---|---|---|
| NB 194 | 1.39E+05 | 7.36E-05 | 5.31E-10 |
| NB 194-P29 | 2.43E+05 | 2.86E-05 | 1.18E-10 |

### Example 12: NB194-P29 humanization

NB194-P29 antibody was humanized by the humanization method described in Example 10, and ten humanized molecules were obtained, all of which had similar affinities to NB194-P29.

| Sample | ka(1/Ms) | kd (1/s) | KD (M) |
|---|---|---|---|
| NB 194-P29 | 2.90E+05 | 4.37E-05 | 1.51E-10 |
| NB194-P29-z1 | 1.17E+05 | 3.06E-05 | 2.63E-10 |
| NB194-P29-z2 | 1.29E+05 | 1.66E-05 | 1.29E-10 |
| NB194-P29-z3 | 1.86E+05 | 3.75E-05 | 2.02E-10 |
| NB194-P29-z4 | 1.17E+05 | 2.61E-05 | 2.22E-10 |
| NB194-P29-z5 | 1.21E+05 | 2.43E-05 | 2.00E-10 |
| NB194-P29-z6 | 1.13E+05 | 3.02E-05 | 2.68E-10 |
| NB194-P29-z7 | 1.45E+05 | 2.18E-05 | 1.50E-10 |
| NB194-P29-z8 | 1.08E+05 | 2.54E-05 | 2.35E-10 |
| NB194-P29-z9 | 1.24E+05 | 5.03E-05 | 4.06E-10 |
| NB194-P29-z10 | 9.68E+04 | 1.74E-05 | 1.79E-10 |

## Claims

1. A PD-1 binding molecule, comprising an anti-PD-1 single domain antibody, wherein the complementarity determining region (CDRs) of the single domain antibody comprise CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3, wherein,
SEQ ID NO: 1 is GX₁X₂X₃X₄X₅X₆X₇, wherein X₁ is D, F, G, H, L or R, X₂ is P, S or T, X₃ is A, F, I, L, N, R, S or V, X₄ is D, F, G, L, M, N or S, X₅ is A, D, F, G, I, N, R, S or T, X₆ is D, F, H, I, L, S, V, W or Y, X₇ is A, D, E, G, N, P, S or T; preferably, SEQ ID NO: 1 is GX₁X₂X₃X₄X₅X₆X₇, wherein X₁ is D, F, G, H, L or R, X₂ is P, S or T, X₃ is F or S, X₄ is For S, X₅ is G, I or T, X₆ is S or Y, X₇ is A or D,
SEQ ID NO: 2 is X₁X₂X₃X₄X₅X₆X₇X₈X₉, wherein X₁ is I, L, S, T or V, X₂ is A, N, S or T, X₃ is F, G, I, K, L, M, N, Q, R, S, T, W or Y, X₄ is A, D, G, H, N, R, SorT, X₅ is A, D, G, N, R, S or null, X₆ is G or R, SorT, X₇ is D, E, I, L, M, N, R, S, T or V, X₈ is A, K, M, Q or T, X₉ is A or null; preferably, SEQ ID NO :2 is X₁X₂X₃X₄X₅GX₇TX₉, where X₁ is I or V, X₂ is N, S or T, X₃ is F, L or N, X₄ is A, G, S or T, X₅ is R or null, and X₇ is D, N , I or T, X₉ is null,
SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is A, E, G, N, R, S, T or V, X₂ is A, G, I, K, L, P, R, T or V, X₃ is A, D, E, G, K, N, P, R, S, V or Y, X₄ is A, C, D, E, G, I, K, L, R, S, T, V or Y, X₅ is A, C, D, F, G, H, I, K, L, N, P, Q, R, S, W or Y, X₆ is A, C, D, E, G, I, K, L, M, P, Q, R, S or Y, X₇ is A, C, D, F, G, H, I, N, P, R, S, T, V or Y, X₈ is A, C, D, E, G, I, R, S, T, V, W, Y or null, X₉ is A, D, E, F, G, I, L, P, R, S, V, W, Y or null, X₁₀ is C, D, F, G, K, L, N, P, R, S, T, V, W, Y or null, X₁₁ is D, F, G, H, I, K, L, N, P, S, T, V, Y or null, X₁₂ is A, D, G, H, I, L, M, P, R, S, T, V, Y or null, X₁₃ is C, D, E, H, P, R, S, T, V, Y or null, X₁₄ is A, D, E, F, H, I, L, P, R, T, Y or null, X₁₅ is A, D, E, G, N, Q, R, S, T, V, Y or null, X₁₆ is A, D, E, F, G, H, M, P, S, V , Y or null, X₁₇ is A, D, E, H, N, Q, R, T, Y or null, X₁₈ is D, E, R, S, Y or null, X₁₉ is N, S, Y or null, X₂₀ is D, E, Y or null, X₂₁ is E, N, Y or null, X₂₂ is Y or null; preferably, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is E, G, N or V, X₂ is A, G, I, L or V, X₃ is A, D, E, G, N, V or Y, X₄ is A, G, I, K, R or S, X₅ is C, I, L, P, Q, R or W, X₆ is E, K, P, Q or R, X₇ is C, D, H, N, P or Y, X₈ is E, G, R, V or W, X₉ is F, G, L, S, V or W, X₁₀ is C, D, G, L, R, S or V, X₁₁ is F, G, I, L, N, S, T or V, X₁₂ is H, L, P, R, S, T or Y, X₁₃ is D, H, P, S, T or V, X₁₄ is E, F, H, I, L, P or R, X₁₅ is A, S or V, X₁₆ is A, D, E, G, H or V, X₁₇ is H, N, Q, R or Y, X₁₈-X₂₂ is null.

2. The PD-1 binding molecule according to claim 1, wherein CDR1 of the single domain antibody comprises the sequence shown in any one of SEQ ID NOs: 4-39 and 320, CDR2 comprises the sequence shown in any one of SEQ ID NOs: 40-75, and CDR3 comprises the sequence shown in any one of SEQ ID NOs: 76-183,
preferably, the single domain antibody comprises CDR1, CDR2 and CDR3 shown in any of the following groups a1 to a136:
| CDR1 | CDR2 | CDR3 | Group |
|---|---|---|---|
| 4 | 40 | 76 | a1 |
| 5 | 41 | 77 | a2 |
| 6 | 42 | 78 | a3 |
| 7 | 43 | 76 | a4 |
| 8 | 44 | 79 | a5 |
| 9 | 45 | 80 | a6 |
| 10 | 46 | 81 | a7 |
| 11 | 47 | 82 | a8 |
| 12 | 48 | 83 | a9 |
| 13 | 49 | 84 | a10 |
| 10 | 50 | 85 | a11 |
| 14 | 47 | 76 | a12 |
| 15 | 51 | 86 | a13 |
| 16 | 52 | 76 | a14 |
| 17 | 53 | 87 | a15 |
| 18 | 54 | 88 | a16 |
| 19 | 55 | 87 | a17 |
| 20 | 56 | 82 | a18 |
| 21 | 57 | 89 | a19 |
| 22 | 58 | 90 | a20 |
| 23 | 59 | 91 | a21 |
| 14 | 47 | 76 | a22 |
| 24 | 60 | 92 | a23 |
| 25 | 40 | 76 | a24 |
| 26 | 61 | 93 | a25 |
| 27 | 62 | 94 | a26 |
| 24 | 60 | 95 | a27 |
| 28 | 41 | 96 | a28 |
| 10 | 63 | 97 | a29 |
| 5 | 41 | 98 | a30 |
| 29 | 64 | 99 | a31 |
| 7 | 65 | 100 | a32 |
| 30 | 66 | 101 | a33 |
| 31 | 67 | 102 | a34 |
| 32 | 68 | 103 | a35 |
| 33 | 56 | 104 | a36 |
| 34 | 69 | 105 | a37 |
| 35 | 70 | 106 | a38 |
| 7 | 71 | 107 | a39 |
| 16 | 52 | 76 | a40 |
| 16 | 52 | 76 | a41 |
| 16 | 52 | 76 | a42 |
| 16 | 52 | 76 | a43 |
| 16 | 52 | 76 | a44 |
| 19 | 72 | 87 | a45 |
| 19 | 72 | 87 | a46 |
| 19 | 72 | 87 | a47 |
| 19 | 73 | 87 | a48 |
| 19 | 53 | 87 | a49 |
| 19 | 53 | 87 | a50 |
| 19 | 53 | 108 | a51 |
| 19 | 53 | 109 | a52 |
| 19 | 53 | 110 | a53 |
| 19 | 53 | 111 | a54 |
| 36 | 74 | 112 | a55 |
| 19 | 53 | 113 | a56 |
| 19 | 53 | 114 | a57 |
| 19 | 53 | 115 | a58 |
| 19 | 53 | 116 | a59 |
| 19 | 53 | 117 | a60 |
| 19 | 53 | 118 | a61 |
| 19 | 53 | 119 | a62 |
| 19 | 53 | 120 | a63 |
| 19 | 53 | 121 | a64 |
| 19 | 53 | 122 | a65 |
| 19 | 53 | 123 | a66 |
| 19 | 53 | 124 | a67 |
| 19 | 53 | 125 | a68 |
| 19 | 53 | 126 | a69 |
| 19 | 53 | 127 | a70 |
| 19 | 53 | 128 | a71 |
| 19 | 53 | 129 | a72 |
| 19 | 53 | 130 | a73 |
| 19 | 53 | 131 | a74 |
| 19 | 53 | 132 | a75 |
| 19 | 53 | 133 | a76 |
| 19 | 53 | 134 | a77 |
| 37 | 75 | 135 | a78 |
| 19 | 53 | 136 | a79 |
| 19 | 53 | 137 | a80 |
| 19 | 53 | 138 | a81 |
| 19 | 53 | 139 | a82 |
| 19 | 53 | 140 | a83 |
| 19 | 53 | 141 | a84 |
| 19 | 53 | 142 | a85 |
| 19 | 53 | 143 | a86 |
| 19 | 53 | 144 | a87 |
| 19 | 53 | 145 | a88 |
| 19 | 53 | 146 | a89 |
| 38 | 53 | 147 | a90 |
| 19 | 53 | 148 | a91 |
| 19 | 53 | 149 | a92 |
| 19 | 53 | 150 | a93 |
| 19 | 53 | 151 | a94 |
| 19 | 53 | 152 | a95 |
| 19 | 53 | 153 | a96 |
| 19 | 53 | 154 | a97 |
| 39 | 53 | 155 | a98 |
| 19 | 53 | 156 | a99 |
| 19 | 53 | 157 | a100 |
| 19 | 53 | 158 | a101 |
| 19 | 53 | 159 | a102 |
| 19 | 53 | 160 | a103 |
| 19 | 53 | 161 | a104 |
| 19 | 53 | 162 | a105 |
| 19 | 53 | 163 | a106 |
| 37 | 53 | 164 | a107 |
| 19 | 53 | 165 | a108 |
| 19 | 53 | 166 | a109 |
| 19 | 53 | 167 | allO |
| 19 | 53 | 168 | a111 |
| 36 | 74 | 169 | a112 |
| 38 | 53 | 170 | a113 |
| 37 | 53 | 171 | a114 |
| 19 | 53 | 172 | a115 |
| 19 | 53 | 173 | a116 |
| 38 | 53 | 174 | a117 |
| 38 | 53 | 175 | a118 |
| 36 | 74 | 176 | a119 |
| 19 | 53 | 177 | a120 |
| 19 | 53 | 178 | a121 |
| 19 | 53 | 179 | a122 |
| 19 | 53 | 180 | a123 |
| 19 | 53 | 181 | a124 |
| 19 | 53 | 182 | a125 |
| 19 | 53 | 183 | a126 |
| 37 | 75 | 135 | a127 |
| 37 | 75 | 135 | a128 |
| 37 | 75 | 135 | a129 |
| 37 | 75 | 135 | a130 |
| 37 | 75 | 135 | a131 |
| 37 | 75 | 135 | a132 |
| 37 | 75 | 135 | a133 |
| 37 | 75 | 135 | a134 |
| 320 | 75 | 135 | a135 |
| 37 | 75 | 135 | a136 |

3. The PD-1 binding molecule according to claim 1 or 2, wherein,
the FR region of the single domain antibody comprises the FR region of any VHH selected from SEQ ID NOs: 184-319, and/or
the single domain antibody VHH is as shown in any one of SEQ ID NOs: 184-319, and/or
the PD-1 binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.

4. The PD-1 binding molecule according to claim 3, wherein the PD-1 binding molecule is a heavy chain antibody, which further comprises a heavy chain constant region,
preferably, the heavy chain constant region comprises the sequence shown in SEQ ID NO: 321.

5. A polynucleotide, wherein the polynucleotide comprises a sequence selected from:
(1) a coding sequence of the PD-1 binding molecule according to any one of claims 1-4;
(2) a complementary sequence of (1);
(3) a 5-50 bp fragment of any sequence of (1) or (2).

6. A nucleic acid construct, wherein the nucleic acid construct comprises the polynucleotide of claim 5,
preferably, the nucleic acid construct is a recombinant vector or expression vector.

7. A phage comprising the PD-1 binding molecules according to any one of claims 1-4,
preferably, the PD-1 binding molecule is displayed on the surface of the phage.

8. A host cell, wherein the host cell:
(1) expresses and/or secrets the PD-1 binding molecules according to any one of claims 1-4; and/or
(2) comprises the polynucleotide according to claim 5; and/or
(3) comprises the nucleic acid construct according to claim 6.

9. A method for producing PD-1 binding molecules, comprising: culturing the host cell according to claim 8 under conditions suitable for producing the PD-1 binding molecules, and optionally purifying the PD-1 binding molecules from the culture.

10. A pharmaceutical composition, comprising the PD-1 binding molecule according to any one of claims 1-4, the polynucleotide according to claim 5, the nucleic acid construct according to claim 6, the phage according to claim 7 or the host cell according to claim 8, and a pharmaceutically acceptable excipient,
preferably, the pharmaceutical composition is used for treating cancer,

11. Use of the PD-1 binding molecule according to any one of claims 1-4 in the preparation of a medicament for the prevention or treatment of a cancer.

12. A kit for detecting PD-1, which is used to evaluate the therapeutic effect of a medicament or diagnose cancer, wherein the kit comprises the PD-1 binding molecule according to any one of claims 1-4, the polynucleotide according to claim 5, the nucleic acid construct according to claim 6, the phage according to claim 7 or the host cell according to claim 8,
preferably, the kit further comprises a reagent for detecting the binding of PD-1 to a single domain antibody, an antibody, or an antigen binding fragment thereof,
more preferably, the reagent is a reagent that detects the binding by enzyme-linked immunosorbent assay.

13. A non diagnostic method for detecting the presence of PD-1 in a sample, wherein the method comprises: incubating a PD-1 binding molecule according to any one of claims 1-4 with the sample, and detecting the binding of PD-1 to a single domain antibody, antibody, or antigen binding fragment thereof, thereby determining the presence of PD-1 in the sample.

14. Use of the PD-1 binding molecule according to any one of claims 1-4 in the preparation of a kit for detecting PD-1 in samples, evaluating the therapeutic effect of a medicament, or diagnosing a cancer.
